Europäisches Patentamt

European Patent Office

Office européen des brevets

(11) Publication number: **0 390 592**
**A2**

## (12) EUROPEAN PATENT APPLICATION

(21) Application number: 90303445.2

(22) Date of filing: 30.03.90

(51) Int. Cl.5: **C12N 15/00, C12N 15/62,**
**C12N 15/58, C12N 15/90**

The microorganism(s) has (have) been deposited with Fermentation Research Institute under number(s) FERM BP-2336, 2335 and 2337.

(30) Priority: **31.03.89 JP 78574/89**

(43) Date of publication of application:
**03.10.90 Bulletin 90/40**

(84) Designated Contracting States:
**AT BE CH DE DK ES FR GB GR IT LI LU NL SE**

(71) Applicant: **KYOWA HAKKO KOGYO KABUSHIKI KAISHA**
**6-1, Ohte-machi 1-chome**
**Chiyoda-ku Tokyo 100(JP)**

Applicant: **CENTRAL INSTITUTE FOR EXPERIMENTAL ANIMALS**
**1430 Nogawa, Miyamae-ku**
**Kawasaki-shi, Kanagawa-ken(JP)**

(72) Inventor: **Sekine, Susumu**
**13-1 Naruse 2-chome**
**Machida-shi, Tokyo(JP)**
Inventor: **Ito, Seiga**
**9-48 Aihara 6-chome**
**Sagamihara-shi, Kanagawa-ken(JP)**
Inventor: **Katsuki, Motoya**
**7-12-703 Chuorinkan 4-chome**
**Yamato-shi, Kanagawa-ken(JP)**

(74) Representative: **Myerscough, Philip Boyd et al**
**J.A.Kemp & Co. 14, South Square Gray's Inn**
**London, WC1R 5EU(GB)**

(54) **Recombinant vector.**

(57) A recombinant vector suitable for industrially making an animal produce useful protein in the milk, which is constructed by extracting high molecular weight DNA from the bovine liver to prepare a chromosomal gene library, preparing a synthetic DNA probe corresponding to the publicly known bovine αS1 casein chromosomal gene, screening a clone carrying bovine αS1 casein chromosomal gene, confirming that the clone has a base sequence coincident with that of the publicly known bovine αS1 casein chromosomal gene, ligating human prourokinase cDNA with the promoter part and the intron 1 part of the gene, and then inserting the obtained plasmid into a suitable vector. A transgenic mouse prepared by using this recombinant vector secreted a considerable amount of human prourokinase as useful protein in its milk after delivery.

FIG. 1

BOVINE αS1 CASEIN CHROMOSOMAL GENE

## RECOMBINANT VECTOR

### BACKGROUND OF THE INVENTION

This invention relates to a recombinant vector in order to make a transgenic animal produce useful protein efficiently, the transgengic animal and a method for producing the useful protein in milk by the animal.

The transduction of an exogenous gene into an embryo of a vertebrate and the gene being inserted into the genome of the aimed animal are publicly known techniques, which are operated according to microinjection method and a method using retrovirus vectors. An animal to be obtained in this operation is called "a transgenic animal". There is a case that this exogenous gene is transmitted to offsprings of the animal according to Mendel's law and is expressed in the offsprings.

Several cases of using a transgenic animal for the production of protein are known. For example, the human blood coagulation factor IX ligated to a metallothionein promoter was expressed in a transgenic mouse and secreted in its blood [Choo et al, *Nucleic Acids Research*, vol. 15, p. 871 (1987)]. The human $\alpha 1$ antitrypsin gene was also expressed in a transgenic mouse and secreted in her blood [Rutter et al, *Nucleic Acids Research*, vol. 15, p. 7519 (1987)]. The sheep $\beta$-lactoglobulin gene was expressed in a transgenic mouse and secreted in its milk [Simons et al, *Nature*, vol. 328, p. 530 (1987)], and a human plasminogen activator ligated to a promoter of whey acidic protein was expressed in mammary gland and secreted in milk [Gordon et al, *Biotechnology*, vol. 5, p. 1183 (1987) and Pittius et al, *Proc. Natl. Acad. Sci.*, U. S. A., vol. 85, p. 5874 (1988)].

As transgenic animals, reports are made not only of mice but also of sheep, pigs, rabbits, etc. [Hammer et al, *Nature*, vol. 315, p. 680 (1985); Simon et al, *Biotechnology*, vol. 6, p. 179 (1988) and Vize et al, *Journal of Cell Science*, vol. 90, p. 295 (1988)]. With respect to the influence of intron upon the expression efficiency in a transgenic mouse, there is a report of recent detail study [Brinster et al, *Proc. Natl. Acad. Sci.*, U.S.A., vol. 85, p. 836 (1988)].

In the conventional method for producing useful protein by the use of gene recombination technique, a system using *Escherichia coli* and a system using a cultured cell have been mainly utilized. In the system using *Escherichia coli*, it is often very difficult to produce the same protein as the original one in case of making the bacterium produce protein of an animal. In case of using a cultured cell, protein which is extremely close to the original one is produced. However, the amount of its production is low, so that this system is unsuitable for the industrial application.

### SUMMARY OF THE INVENTION

The present inventors made studies of a method for effectively producing useful protein. As the result, they succeeded in making a transgenic mouse secrete considerable amounts of useful protein, e.g., human prourokinase in its milk. That is, the present inventors extracted high molecular weight DNA from a bovine liver to prepare a chromosomal gene library. Then, they prepared a synthetic DNA probe corresponding to the publicly known bovine $\alpha S1$ casein chromosomal gene to screen about 150,000 clones carrying the bovine $\alpha S1$ casein genes. As the result, they obtained a clone carrying the bovine $\alpha S1$ casein gene and confirmed that the base sequence thereof was coincident with the publicly known one [Yu-Lee et al, *Nucleic Acids Research*, vol. 14, p. 1883 (1986)]. Then, they ligated the promoter and the first intron part of the clone to a human prourokinase cDNA and inserted the same into a suitable vector. As the result of preparing transgenic mice using the recombinant DNA, they confirmed that, in case of female individuals therof, considerable amounts of human prourokinase was secreted in milk after delivery.

There has been no report that bovine $\alpha S1$ casein chromosomal gene was used for the regulation of gene expression at the time of making a transgenic animal secrete and produce heterologous protein in its milk. It was the present invention by which the utilizability of the bovine $\alpha S1$ casein chromosomal gene for the secretion of heterologous protein in milk was first confirmed.

The object of the present invention lies in providing recombinant vectors which enables a transgenic animal to produce useful protein efficiently, the transgenic animal and a method for making the transgenic animal produce useful protein in its milk.

According to the present invention, it becomes possible to efficiently produce useful protein in milk of an animal.

BRIEF DESCRIPTION OF THE DRAWINGS

Fig. 1 shows a restriction enzyme map of bovine α-S1 casein chromosomal gene.

Fig. 2 shows a construction process of plasmids pBAS1H-S and pBAS1HXS.

Fig. 3 shows a construction process of plasmid pUKGpro1.

Fig. 4 shows a construction process of plasmid pSE1UKpro1-2.

Fig. 5 shows a construction process of plasmid pCUO.

Fig. 6 shows a construction process of plasmids pCUW and pCUT.

Fig. 7 shows one example of the results of Southern blot hybridization using transgenic mouse tail DNA carrying sequences of pCUO and pCUW.

Fig. 8 (1) and (2) show a synthesis process of a cDNA and a recombinant plasmid containing the cDNA according to the Okayama-Berg method.

Fig. 9 shows a construction process of plasmid pCCK1.

Fig. 10 shows a construction process of plasmid pCCK2.

Fig. 11 shows a construction process of plasmid pUK11 conveying human pro-UKcDNA.

Fig. 12 shows a construction process of plasmid pTrS20.

Fig. 13 shows a construction process of plasmid pTrS33.

Fig. 14 shows a construction process of plasmid pTerm2.

Fig. 15 shows a construction process of plasmid pTSF10.

Fig. 16 shows a construction process of plasmid pTA4.

Fig. 17 shows a construction process of plasmid pAGE105M.

Fig. 18 shows a construction process of plasmid pAGE106.

Fig. 19 shows a construction process of plasmid pSE1PA1-5.

Fig. 20 shows a construction process of plasmid pSE1PA1-9.

Fig. 21 shows a construction process of plasmid pUC19H.

Fig. 22 shows a construction process of plasmid pSE1PA1-9A.

Fig. 23 shows a construction process of plasmid pUKA2.

Fig. 24 shows a construction process of plasmid pUKF2.

Fig. 25 shows a construction process of plasmid a pUKFpro.

DETAILED DESCRIPTION OF THE INVENTION

As previously described, this invention contempltes providing a recombinant vector for making a transgenic animal produce useful protein efficiently, the transgenic animal and a method for making the transgenic animal produce useful protein in its milk. In further detail, this invention contemplates providing a method for producing human prourokinase which comprises the steps of introducing a DNA fragment containing a promoter of bovine αS1 casein chromosomal gene into an embryo of an animal, developing the embryo to grow into a matured animal, inducing the animal or female offsprings of the animal to secrete milk containing human prourokinase, collecting milk of the animal in milk secretion stage and collecting human prourokinase from the milk.

All the recombinant DNA manupilation to be used in this invention can be carried out according to common techniques which are generally put into practice [e.g., Maniatis et al, *"Molecular Cloning: A Laboratory Manual"*, Cold Spring Harbor Laboratory (1982)].

Hereinafter, the above production method will be described in detail.

A bovine αS1 casein chromosomal gene is obtained by partially digesting bovine liver high molecular weight DNA with a restriction enzyme such as *Sau* 3A or the like, ininserting the partially digested DNA into an appropriate phage vector to prepare a chromosonal gene library and then screening the library by using a synthetic DNA fully or partially corresponding to the known bovine αS1 casein cDNA as a probe.

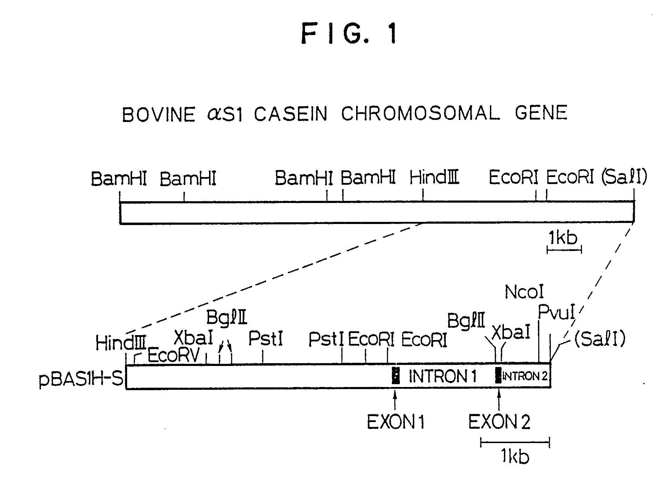

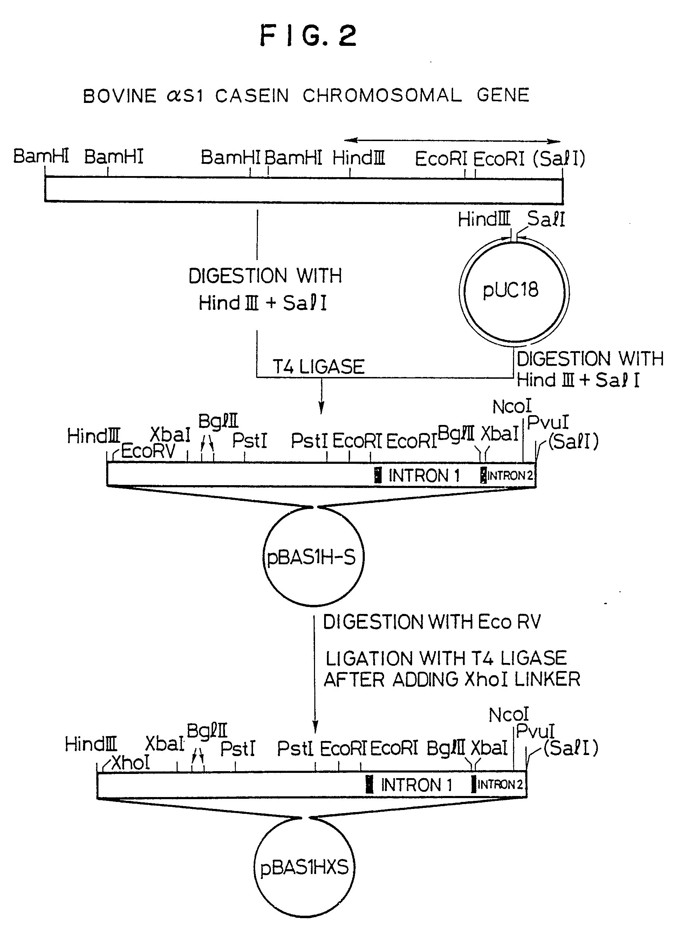

With respect to the bovine αS1 casein cDNA, the base sequences thereof have been already reported [Stewart et al, *Nucleic Acids Research*, vol. 12, p. 3895 (1984); Maki et al, *Agric. Biol. Chem.*, vol 49, p. 1099 (1985)]. In addition, with respect to the bovine αS1 casein chromosomal gene, there is a report made of partial base sequences alone (approx. 1 kb) [Yu-Lee et al, *Nucleic Acids Research*, vol. 14, p. 1883 (1986)]. The isolated bovine αS1 casein chromosomal gene of the present invention has an overall length of 14.5 kb, a restriction enzyme map of which is shown in Fig. 1. The gene is novel in respect of its containing base sequences longer than the foregoing reported partial base sequences by approx. 11 kb upstream and approx. 2 kb down stream, particularly in respect of its containing the whole region of intron 1 and exon 2 and intron 2 halfway. In the bovine αS1 casein chromosomal gene, exon 1 contains 53 bp of non-coding region alone from the transcription initiation site and exon 2 contains 12 bp of non-coding region and 51 bp (corresponding to 17 amino acids, out of which 15 amino acids constitute a signal peptide). Both of exon 1 and exon 2 are very important regions for transcription and translation. The regions are expected to have great influences upon the expression in animal cells, so that it is desired that the regions are contained in a recombinant vector to be expressed. Approx. 6-kb *Hind* III-*Sal* I fragment from the promoter region to the intron 2 of this bovine αS1 casein chromosomal gene is subcloned between *Hind* III-*Sal* I of pUC 18 (e.g., manufactured by Takara Shuzo Co., Ltd.) to obtain pBAS1H-S. Then, *Xho* I linkers are inserted into the digested *Eco* RV site of the plasmid to convert the *Eco* RV site to the *Xho* I site. The obtained plasmid is named pBAS1HXS. This construction process is shown in Fig. 2.

As a human prourokinase gene, both cDNAs and chromosomal genes can be used. As preferable examples thereof, pUKFpro can be enumerated specifically. The construction process of pUKFpro is fully described in Referential Examples 9 and 10. Then, a 1,337-bp fragment obtained by digesting pUKFpro with *Hind* III, and *Bam* HI and a 117-bp fragment obtained by digesting pUKFpro with *Bam* HI and *Pvu* II are re covered and then the both fragments are cloned into pUC19 (e.g., manufactured by Takara Shuzo Co., Ltd.). Thus obtained plasmid was named pUKGpro1.

Furthermore, a splicing signal and a poly A signal are added to human prourokinase gene. Both signals are considered exerting influences upon the expression in eucaryotic cells, so that it is desired that the both signals are added to genes to be expressed. As plasmids to be used, any plasmid will do as far as it contains the signals. As a preferable example, pSE1PA1-5 can be enumerated specifically. The construction process of the pSE1PA1-5 is fully described in Referential Examples 1 to 8. The pSE1PA1-5 is preferable in that a splicing signal and a poly A signal of rabbit β globin and a poly A signal of SV40 are contained in the downstream of human prourokinase gene, particularly in that two poly A signals are ligated thereto. A fragment containing a splicing signal and a poly A signal obtained by digesting pSE1PA1-5 with *Kpn* I and *Hind* III and a fragment containing a human prourokinase gene obtained by digesting pUKGpro1 with *Kpn* I and *Hind* III are linked to obtain pSE1UKpro1-2.

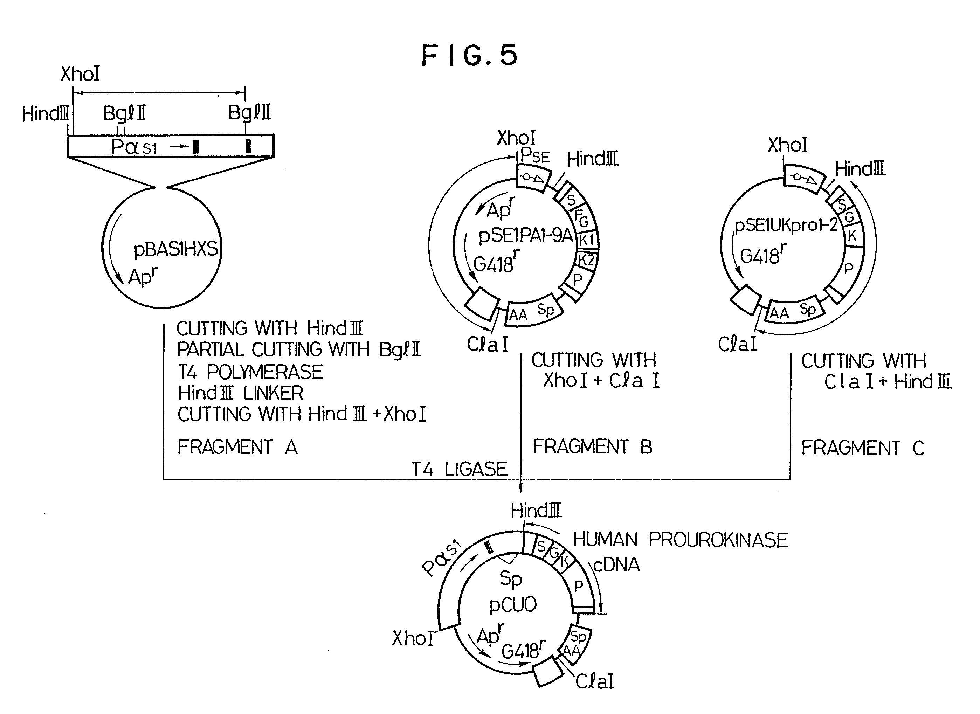

Then, a human prourokinase gene is ligated to the downstream of intron 1 of a bovine αS1 casein chromosomal gene and, simultaneously, an ampicillin-resistance gene is introduced into a plasmid. This costruction process is shown in Fig. 5.

Firstly, the aforementioned pBAS1HXS is completely digested with *Hind* III, followed by partial digestion with *Bgl* II. Subsequently, after blunting the ends of thus treated pBAS1HXS with T4 polymerase, a *Hind* III linker is added thereto and then digested with *Hind* III and *Xho* I to thereby isolate a fragment containing the whole region of intron 1 from the 5´-franking region. This fragment will be referred to as "Fragment A".

Then, pSE1PA1-9A is cut with *Xho* I and *Cla* I to isolate a fragment containing ampicillin-resistance gene and G418-resistance gene. This fragment will be referred to as "Fragment B". A microorganism containing pSE1PA1-9A is deposited with the Fermentation Research Institute (often abbreviated to "Bikoken"), Agency of Industrial Science and Technology as "*Escherichia coli* EhPA1-9A" on September 3, 1987 (Accession No. FERM BP-1460). The construction process of the pSE1PA1-9A is fully described in Referential Examples 1 to 8.

Then, the aforementioned pSE1UKpro1-2 is cut with *Hind* III and *Cla* I to isolate a fragment containing human prourokinase gene, a splicing signal and a poly A signal. This fragment will be referred to as "Fragment C".

The above Fragments A, B and C are linked with a T4 ligase to obtain pCUO.

The pCUO contains approx. 3.6 kb ranging from the 5´-end of exon 1 to the upstream *Eco* RV site (which is modified to *Xho* site), exon 1 and intron 1 of bovine αS1 casein chromosomal gene and, just behind the same, 76bp of 5´-non-coding region, the whole coding region and 76bp of 3´-non-coding region of human prourokinase gene cDNA, a splicing signal and a poly A signal of rabbit β-globin gene, a poly A signal of SV40, G418-resistance gene and ampicillin-resistance gene.

Then, a *Hind* III-*Kpn* I fragment (Fragment D) containing bovine αS1 casein chromosonal gene ranging from the upper stream of the 5´-end to intron 1, ampicillin-resistance gene, G418-resistance gene, a splicing signal and a poly A signal of rabbit β-globin gene and a poly A signal of SV40, a *Taq* I-*Pst* I fragment (Fragment E) containing a growth factor domain and a part of a cringle of human prourokinase gene cDNA and a *Pst* I-*Kpn* I fragment (Fragment F) containing a region behind the cringle are isolated from the pCUO. Furthermore, a synthetic DNA (Linker 1) corresponding to the signal peptide of bovine αS1 casein and the N-terminal nine amino acids of mature human prourokinase and a synthetic DNA (Linker 2) composed of an arginine codon (AGG) being inserted in the ligation site of the signal peptide and the mature human prourokinase are prepared, which are shown in next page.

**Linker 1**

*Hind* III

Initiation →

Bovine αS1 Casein Signal Peptide

```
5'- AGCTTGACAACC ATGAAACTTCTTATCCTCACGTGTCTTGTGGCTGTTGCTCTTGCC
3'- ACTGTTGG    TACTTTGAAGAATAGGAGTGCACAGAACACCGACAACGAGAACGG
               MetLysLeuIleLeuThrCysLeuValAlaValAlaAlaLeuAla
```

Mature Human → Prourokinase

*Taq* I

```
AGCAATGAACTTCATCAAGTTCCAT      -3'
TCGTTACTTGAAGTAGTTCAAGGTAGC    -5'
SerAsnGluLeuHisGlnValProSer
```

**Linker 2**

*Hind* III

Initiation →

Bovine αS1 Casein Signal Peptide

```
5'- AGCTTGACAACC ATGAAACTTCTTATCCTCACGTGTCTTGTGGCTGTTGCTCTTGCC AGG
3'- ACTGTTGG    TACTTTGAAGAATAGGAGTGCACAGAACACCGACAACGAGAACGG  TCC
               MetLysLeuIleLeuThrCysLeuValAlaValAlaAlaLeuAla    Arg
```

Mature Human → Prourokinase

*Taq* I

```
AGCAATGAACTTCATCAAGTTCCAT      -3'
TCGTTACTTGAAGTAGTTCAAGGTAGC    -5'
SerAsnGluLeuHisGlnValProSer
```

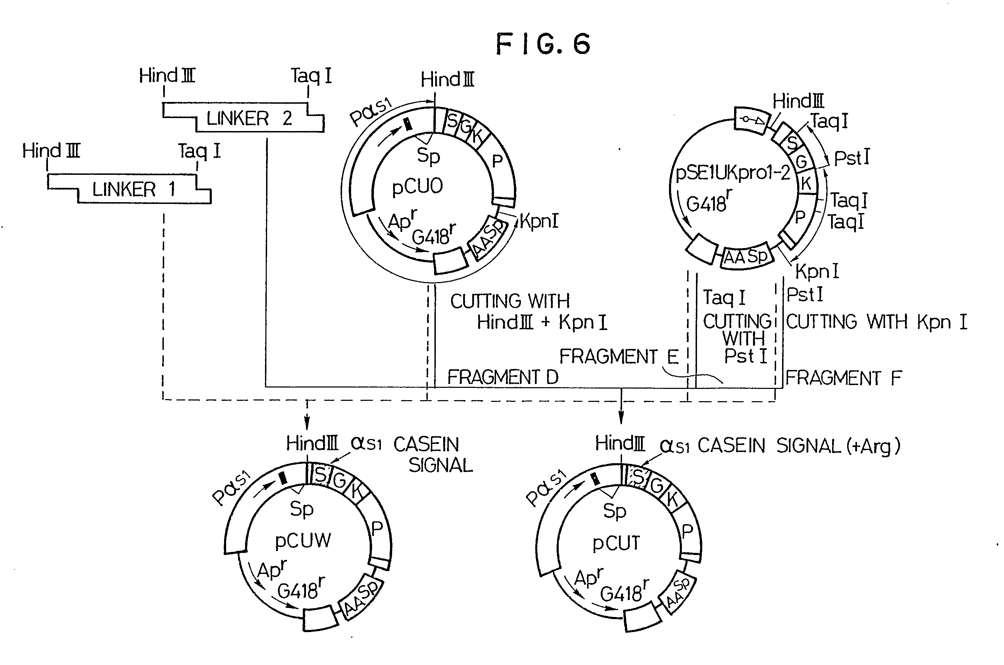

The aforementioned Fragments D, E and F are ligated with Linkers 1 and 2 to obtain pCUW and pCUT respectively. The construction processes of pCUW and pCUT are shown in Fig. 6. The pCUO, pCUW and pCUT have very close structure. However, they are respectively featured that the signal peptide part which plays an important role in the secretion process of protein is derived from human prourokinase in case of the pCUO and from bovine αS1 casein chromosomal gene in case of the pCUW and that arginine (Arg) is inserted between signal peptide of bovine αS1 casein chromosomal gene and mature human prourokinase in case of pCUT. By the insertion of Arg, a link of casein signal - casein N-terminal Arg - mature proUK is formed and the signal-Arg site is liable to be cut. Accordingly, it is expected that the separtion of the signal and the mature proUK is carried out more efficiently. As will be described later, it is supposed to be effective for the expression in the mammary gland to use the signal peptide of gene expressed in the mammary gland in a natural state. Thus, the signal sequences of bovine αS1 casein chromosomal gene used here is is desirable.

Then, *Xho* I-*Cla* I fragments ranging from bovine αS1 chromosomal gene to the poly A signal of SV40 are recovered from the pCUO, pCUW and pCUT and then injected into nuclei of pronucleus-stage eggs of mice to obtain transgenic mice. The preparation method of a transgenic mouse in this invention is based on the method fully described in "How to prepare a transgenic mouse" in *A Laboratory Manual of Embryological Engineering* [Tatsuji Nomura (supervised), Motoya Katsuki (ed.), Kodansha Scientific (1987)-

]. Among the obtained transgenic mice, one individual carrying pCUO (named CUO7-6ℓ) and another one individual carrying pCUW (named CUW13-1ℓ) respectively secrete approx. 300ng/mℓ of prourokinase in their milk. The milk is subjected to acid treatment (e.g, at pH 4.5), and casein is precipitated and removed. Then, the pH of thus treated milk is brought back to about 7.5. The resulting milk is dialyzed against an appropriate buffer solution and then passed through a column packed with anti-human prourokinase antibody-bound Sepharose to make human prourokinase adsorb thereon. Thereafter, the adsorbed human prourokinase is eluted with a buffer solution of pH 4.0, purified and then recovered. Prourokinase is hardly observed in the milk of common mouse individuals, so that it is obvious that human prourokinase was specifically expressed in transgenic mice carrying the sequences of pCUO and pCUW.

Strains of *Escherichia coli* containing pCUO, pCUW and pCUT were deposited with the Fermentation Research Institute as *Escherichia coli* ECUO, *Escherichia coli* ECUW *and Escherichia coli* ECUT with accession numbers FERM BP-2336, -2335 and -2337 respectively on March 14, 1989.

The present invention also provides the following recombinant vectors:

(1) A recombinant vector in which a promoter of bovine αS1 casein chromosomal gene and human prourokinase gene are integrated in a vector.

(2) A recombinant vector in which a promoter of bovine αS1 casein chromosomal gene, human prourokinase gene, a splicing signal and a poly A signal of rabitt β-globin gene and a poly A signal of SV40 are integrated in a vector.

(3) A recombinant vector in which a promoter of bovine α-S1 casein chromosomal gene, human prourokinase gene, a splicing signal and a poly A signal of rabbit β-globin gene, a poly A signal of SV40 and ampicillin-resistance gene and/or G418-resistance gene are integrated in a vector.

(4) A recombinant vector in which a promoter of bovine αS1 casein chromosomal gene, a splicing signal of bovine αS1 casein chromosomal gene, human prourokinase gene, a splicing signal and a poly A signal of rabbit β-globin, a poly A signal of SV40 and ampicillin-resistance gene and/or G418-resistance gene are integrated in a vector.

(5) Plasmid pCUO represented by the restriction map shown in Fig. 5 and contained in *Escherichia coli* ECUO (FERM BP-2336).

(6) Plasmid pCUW represented by the restriction map shown in Fig. 6 and contained in *Escherichia coli* ECUW (FERM BP-2335).

(7) Plasmid pCUT represented by the restriction map shown in Fig. 6 and contained in *Escherichia coli* ECUT (FERM BP-2337).

Respective elements and production methods of these recombinant vectors are as described in the above production method of human prourokinase.

The present invention still also provides the following animals:

(1) An animal in which a promoter of bovine αS1 casein chromosomal gene and human prourokinase gene are integrated in its nucleus.

(2) An animal in which a promoter of bovine αS1 casein chromosomal gene and human prourokinase gene are integrated in its chromosome.

As the animals, a sheep, a pig, a goat, cattle and a mouse are used. As the promoter of bovine αS1 casein chromosomal gene and the human prourokinase gene to be integrated, those derived from the above plasmids pCUO, pCUW and pCUT are used preferably.

As a method for integrating a gene in an animal, a method described in "How to prepare a transgenic mouse" in *A Laboratory Manual of Embryological Engineering* [Tatsuji Nomura (supervised), Motoya Katsuki (ed.), Kodansha Scien tific (1987)] is used.

EXAMPLES

Hereinafter, the present invention will be described, referring to examples.

Example 1

Isolation of Bovine αS1 Casein Chromosomal Gene

Approx. 10g of fresh bovine liver was disrupted in 100mℓ of solution containing 10mM Tris-HCℓ (pH

8.0), 100mM NaCl and 10mM EDTA by using a whirling blender (a power homogenizer manufactured by Nippon Seiki Co., Ltd.) (100 to 1,000 rpm, 30 sec.), followed by addition of Proteinase K (manufactured by Sigma Chemical Co.) and sodium dodecyl sulfate (SDS) respectively to final concentrations of 200μg/ml and 0.5%. The resulting solution was allowed to stand for 4 hours at 65°C. The solution was extracted twice with phenol and once with phenol-chloroform (1:1), followed by addition of NaCl to give a final concentration of 200mM. Then, 2-fold volume of ethanol was added to the resulting solution to precipitate DNA. The DNA was recovered by fishing out with a glass rod, washed with 80% ethanol and then dried under reduced pressure by using a vacuum pump. Whereby, approx. 22mg of high molecular weight DNA was obtained.

300μg of the high molecular weight DNA was dissolved in 2ml of solution containing 10mM Tris-Hcl - (pH 7.5), 7mM MgCl$_2$ and 100mM NaCl. Then, 4.4 units of $Sau$ 3AI (manufactured by Takara Shuzo Co., Ltd.; enzymes to be used hereinafter are all manufacured by Takara Shuzo Co., Ltd. unless otherwise referred to particularly) was added to the above DNA solution to carry out partial digestion reaction for 1 hour at 37°C. The reaction solution was subjected to 10~40%-sucrose density gradient centrifugation (an RPS-40T rotor manufactured by Hitachi, Ltd.; 28krpm, 18 hours). After fractionating the reaction solution by 0.5ml portions, fractions rich in approx. 20-kb fragments were dialyzed and recovered by ethanol precipitation. Whereby, approx. 8μg of 20-kb fragment was obtained.

On the other hand, phage vector EMBL 3 (manufactured by Toyobo Co., Ltd.) was prepared according to an ordinary method [see "*Molecular Cloning: A Laboratory Manual*", by Maniatis et al, pp. 77~85, Cold Spring Harbor Laboratory (1982)], out of which 50μg was dissolved in 1ml of solution containing 10mM Tris-HCl (pH 7.5), 7mM MgCl$_2$ and 100mM NaCl. Then, 100 units of $Bam$ HI was added to the solution to carry out digestion reaction for 15 hours at 37°C. The reaction solution was subjected to the aforementioned sucrose density gradient centrifugation to recover fractions containing EMBL3 vector abundantly. Whereby, approx. 12μg of EMBL3 vector can be obtained.

1μg of the vector DNA and approx. 3μg of the aforementioned 20-kb chromosome fragment were dissolved in 20μl of solution containing 66mM Tris-HCl (pH 7.6), 6.6mM MgCl$_2$, 10mM DTT and 1mM ATP. Then, approx. 150 units of T4 DNA ligase was added to the solution to carry out ligation reaction for 24 hours at 4°C. The reaction solution was ethanol precipitated to recover DNA, followed by packaging of the the DNA by mixing the same with a reaction solution of an *in vitro* packaging kit (manufactured by Toyobo Co., Ltd.). Using the reaction solution, approx. 150,000 plaques were formed on agar plates according to an ordinary method [see "*Molecular Cloning: A Laboratory Manual*", Maniatis et al, pp. 320~321, Cold Spring Harbor Laboratory (1982)]. These plaques were screened by using 2 types of $^{32}$P-labelled synthetic DNA porbes according to plaque hybridization to thereby obtain one positive clone. The synthetic probes used are shown below.

Probe 1
5′-TCACTTCGACCATCAACCCAGCTTGCTGTTCTTCCCAGTC-3′
Probe 2
5′-ATGAAACTTCTCATCCTTACCTGTCTTGTGGCTGTTGCTC-3′

These probes were prepared by using a 380A-DNA synthesizer manufactured by Applied Bio Systems, Inc.

The sequence of Probe 1 corresponds to the 5′-non-coding region of bovine αS1 casein cDNA [Maki et al, *Agric. Biol. Chem.*, vol. 49, p. 1099 (1985)] and a part of exon 1 of bovine αS1 case in chromosomal gene (Yu-Lee et al, *Nucleic Acids Research*, vol. 14, p. 1883 (1986)]. On the other hand, Probe 2 corresponds to base sequences of a region encoding a signal peptide of bovine αS1 casein cDNA.

The aforementioned positive clone was confirmed that it hybridized to both of Probes 1 and 2 also by Southern blot hybridization. The restriction enzyme map of the clone was shown in the upper part of Fig. 1.

Example 2

Subcloning of Region from Promoter to Intron 1 of Bovine αS1 Casein Chromosomal Gene

5μg of approx. 14.5-kb DNA containing the bovine αS1 casein chromosomal gene described in Example 1 was dissolved in 40μl of solution containing 10mM Tris-HCl (pH 7.5), 7mM MgCl$_2$ and 100mM NaCl. Then, each 10 units of *Hind* III and *Sal* I were added to the solution to carry out digestion reaction for 3 hours at 37°C. The reaction solution was subjected to agarose gel electrophoresis to isolate and recover fragments of approx. 6 kb ranging from a promoter to intron 2. Whereby, approx. 0.1μg of fragment

was obtained.

Then, 2μg of pUC18 was dissolved in 40μl of solution 10mM Tris-HCl (pH 7.5), 7mM MgCl₂ and 100mM NaCl, to which each 5 units of Hind III and Sal I were then added to carry out digestion reaction for 2 hours at 37°C. The reaction solution was subjected to agarose gel electrophoresis to isolate and recover fragments of approx. 2.7 kb. Whereby, approx. 1μg of the fragment was obtained.

0.1μg of the above 6-kb bovine αS1 casein chromosomal gene fragment and 0.05μg of the above pUC18 were dissolved in 30μl of solution containing 66mM Tris-HCl (pH 7.6), 6.6mM MgCl₂, 10mM dithiothreitol (DTT) and 1mM ATP, followed by addition of approx. 150 units of T4 DNA ligase to carry out ligation reaction for 24 hours at 4°C. Escherichia coli strain MM294 (F⁻ hsdR⁻ hsdM⁺ endoI⁻ thi) [ATCC31446; the same strain as Escherichia coli strain 294 given in a paper "K. Beckman et al, Proc. Natl. Acad. Sci., U.S.A., vol. 73, p. 4171 (1976)"] was transformed by the reaction solution to thereby obtain an ampicillin-resistance strain.

Plasmid DNA was recovered from this transformed strain and subjected to restriction enzyme analysis to obtain pBAS1H-S (see Fig. 2).

Then, 1μg of pBAS1H-S was dissolved in 40μl of solution containing 10mM Tris-HCl (pH 7.5), 7mM MgCl₂ and 150mM NaCl to which 5 units of Eco RV was then added to carry out digestion reaction for 2 hours at 37°C. The reaction solution was subjected to agarose gel electrophoresis to isolate and recover a fragment of approx. 8.7 kb. Whereby, 0.4μg of the fragment was obtained.

Separately, 0.2μg of Xho I linker (manufactured by Takara Shuzo Co., Ltd.) was dissolved in 20μl of solution containing 50mM Tris-HCl (pH 7.6), 10mM MgCl₂, 10mM mercaptoethanol and 1mM ATP, followed by addition of 5 units of T4 polynucleotide kinase to carry out phosphorylation reaction for 30 minutes at 37°C. 1μl of the reaction solution and the above 8.7-kb fragment of pBAS1H-S were dissolved in 30μl of solution containing 66mM Tris-HCl (pH 7.6), 6.6mM MgCl₂, 10mM DTT and 1mM ATP, followed by addition of approx. 150 units of T4 DNA ligase to carry out ligation reaction for 24 hours at 4°C. Escherichia coli strain MM294 was transformed by using the reaction solution to obtain an ampicillin-resistance strain. Plasmid DNA was recovered from this transformed strain and then subjected to restriction enzyme analysis to obtain pBAS1HXS. This plasmid was a transformant in which Eco RV site near of the 5′-end of bovine αS1 casein chromosomal gene of pBAS1H-S was converted to Xho I site (see Figs. 1 and 2).

Example 3

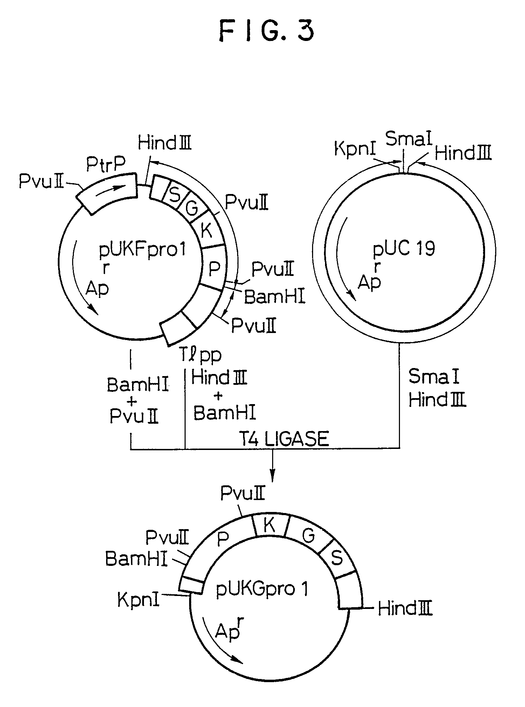

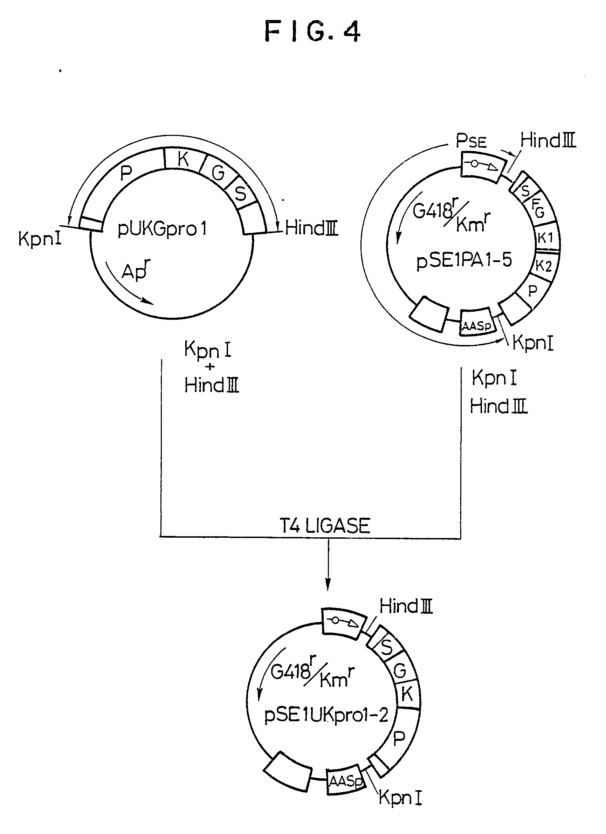

Construction of Plasmid pSE1UKpro1-2 Carrying Splicing Signal and Poly A Signal of Rabbit β-Globin and Poly A Signal of SV40 on Downstream of Human Prourokinase cDNA (see Figs. 3 and 4)

5μg of plasmid pUKFpro containing human prourokinase (construction process thereof was described in Referential Examples 9 and 10) was dissolved in 40μl of solution containing 10mM Tris-HCl (pH 7.5), 7mM MgCl₂ and 100mM NaCl, followed by addition of each 10 units of Bam HI and Hind III to carry out digestion reaction for 2.5 hours at 37°C. The reaction solution was subjected to agarose gel electorphoresis to islate and recover a 1,337-bp fragment. Whereby, approx. 1μg of the fragment was obtained.

Separately, 20μg of pUKFpro was dissolved in 40μl of solution containing 10mM Tris-HCl (pH 7.5), 7mM MgCl₂ and 100mM NaCl, followed by addition of each 25 units of Bam HI and Pvu II to carry out digestion reaction for 2 hours at 37°C. The reaction solution was subjected to agarose gel electrophoresis to isolate and recover 0.1μg of 117-bp fragment.

Separately, 5μg of pUC19 was dissolved in 20μl of solution containing 10mM Tris-HCl (pH 8.0), 7mM MgCl₂ and 20mM KCl, followed by addition of 10 units of Sma I to carry out digestion reaction for 2 hours at 37°C. Then, NaCl was added to the reaction solution to give a final concentration of 60mM, followed by addition of 10 units of Hind III to further carry out digestion reaction for 2 hours. The reaction solution was subjected to agarose gel electrophoresis to isolate and recover a fragment of approx. 2.7 kb. Whereby, 2 μg of the fragment was obtained.

0.4μg of Hind III-Bam HI fragment and 0.05μg of Bam HI-Pvu II fragment of pUKFpro and 0.1μg of Sma I-Hind III fragment of pUC19 obtained above were dissolved in 30μl of solution containing 66mM Tris-HCl (pH 7.6), 6.6mM MgCl₂, 10mM DTT and 1mM ATP, followed by addition of approx. 150 units of T4 DNA ligase to carry out ligation reaction for 20 hours at 4°C. Escherichia coli strain MM294 was transformed by using the reaction solution to obtain an ampicillin-resistance strain. Then, plasmid DNA was recovered from this transformed strain and subjected to restriction enzyme analysis to obtain pUKGpro1 (see Fig. 3, wherein S, G, K and P respectively corresponds to domains of a signal peptide, growth factor, cringle and protease of human prourokinase).

Then, 5μg of pUKGpro1 obtained above was dissolved in 40μl of solution containing 10mM Tris-HCl - (pH 7.5) and 7mM MgCl₂, followed by addition of 10 units of *Kpn* I to carry out digestion reaction for 2 hours at 37°C. NaCl was added to give a final concentration of 100mM, followed by addition of 10 units of *Hind* III to further carry out digestion reaction for 2 hours at 37°C. The reaction solution was subjected to agarose gel electrophoresis to isolate and recover a fragment of approx. 1,450 bp containing human prourokinase cDNA. Whereby, 0.8μg of the fragment was obtained.

On the other hand, 5μg of pSE1PA1-5 (construction process thereof was described in Referential Examples 1 to 8) was dissolved in 40μl of solution containing 10mM Tris-HCl (pH 7.5) and 7mM MgCl₂, followed by addition of 10 units of *Kpn* I to carry out digestion reaction for 4 hours at 37°C. Then, NaCl was added to the reaction solution to give a final concentration of 100mM, followed by addition of 10 units of *Hind* III to carry out digestion reaction for 2 hours at 37°C. The reaction solution was subjected to agarose gel electrophoresis to isolate and recover a fragment of approx. 4.8 kb. Whereby, approx. 2μg of the fragment was obtained.

0.5μg of *Kpn* I-*Hind* III fragment of the above pUKGpro1 and 0.2μg of *Kpn* I-*Hind* III fragment of the above pSE1PA1-5 were dissolved in 30μl of solution containing 66mM Tris-HCl (pH 7.6), 6.6mM MgCl₂, 10mM DTT and 1mM ATP, followed by addition of approx. 150 units of T4 DNA ligase to carry out ligation reaction for 24 hours at 4°C. *Escherichia coli* strain MM294 was transformed by using the reaction solution to obtain a kanamycin-resistance strain. Plasmid DNA was recovered from this transformed strain and then subjected to restriction enzyme analysis to obtain pSE1UKpro1-2 (see Fig. 4, wherein Sp and A respectively indicate rough locations of a splicing signal and a poly A signal, as will be adopted hereinafter).

Example 4

Construction of Plasmid pCUO Carrying Human Prourokinase cDNA Downstream of Intron 1 of Bovine αS1 Casein Chromosomal Gene (See Fig. 5)

100μg of pBAS1HXS obtained in Example 2 was dissolved in 200μl of solution containing 10mM Tris-HCl (pH 7.5), 7mM MgCl₂ and 60mM NaCl, followed by addition of 150 units of *Hind* III to carry out digestion reaction for 1 hour at 37°C. The reaction solution was dispensed in 40μl portions, to each of which 11, 9, 7, 5 and 3 units of *Bgl* II was added to carry out *Bgl* II-partial digestion for 1 hour at 37°C. The reaction solution was subjected to agarose gel electorphoresis to isolate and recover a fragment of approx. 5.2 kb. Whereby, approx. 0.5μg of the fragment was obtained in total. Subsequently, the above approx. 0.5μg of fragment was dissolved in 30μl of solution containing 67mM Tris-HCl (pH 8.8), 6.7mM MgCl₂, 10mM mercaptoethanol, 16.6mM (NH₄)₂SO₄, 6.7μM EDTA and each 1mM dATP, dGTP, dCTP and dTTP, followed by addition of 2 units of T4 DNA polymerase to carry out end-filling reaction for 1 hour at 37°C. After phenol extraction, the DNA was recovered by ethanol precipitation.

On the other hand, 0.25μg of *Hind* III linker (manufactured by Takara Shuzo Co., Ltd.) was dissolved in 20μl of solution containing 50mM Tris-HCl (pH 7.6), 10mM MgCl₂, 10mM mercaptoethanol and 1mM ATP, followed by addition of 5 units of T4 polynucleotide kinase to carry out phosphorylation reaction for 30 minutes at 37°C. 2μl of the reaction solution and the whole amount of the aforementioned polymerase-treated pBAS1HXS were dissolved in 30μl of solution containing 66mM Tris-HCl (pH 7.6), 6.6mM MgCl₂, 10mM DTT and 1mM ATP, followed by addition of approx. 150 units of T4 DNA ligase to carry out ligation reaction for 18 hours at 4°C. The reaction solution was phenol extracted and then ethanol precipitated to recover DNA. The whole amount of the recovered DNA was dissolved in 40μl of solution containing 10mM Tris-HCl (pH 7.5), 7mM MgCl₂ and 100mM NaCl, followed by adaddition of each 5 units of *Hind* III and *Xho* I to carry out digestion reaction for 2 hours at 37°C. The reaction solution was subjected to agarose gel electrophoresis to isolate and recover a fragment of approx. 5.2 kb (Fragment A). Whereby, 0.1μg of the fragment was obtained.

Then, 2μg of pSE1PA1-9A [a microorgnism containing this plasmid was deposited with the Fermentation Research Institute as *Escherichia coli* EhPA1-9A on September 3, 1987 (accession No. FERM BP-1460; and construction process thereof was described in Referential Examples 1 to 8)] was dissolved in 40μl of solution containing 10mM Tris-HCl (pH 7.5), 7mM MgCl₂ and 100mM NaCl, followed by addition of each 5 units of *Xho* I and *Cla* I to carry out digestion reaction for 2.5 hours at 37°C. The reaction solution was subjected to agarose gel electrophoresis to isolate and recover a fragment of approx. 4.3 kb (Fragment B). Whereby, approx. 1μg of the fragment was obtained.

Furthermore, 4μg of pSE1UKpro1-2 obtained in Example 3 was dissolved in 40μℓ of solution containing 10mM Tris-HCℓ (pH 7.5), 7mM MgCℓ₂ and 100mM NaCℓ, followed by addition of each 10 units of *Hind* III and *Cla* I to carry out digestion reaction for 24 hours at 37°C. The reaction solution was subjected to agarose gel electrophoresis to isolate and separate a fragment of 3.2 kb (Fragment C). Whereby, approx. 1μg of the fragment was obtained.

0.1μg of Fragment A of pBAS1HXS, 0.1μg of Fragment B of pSE1PA1-9A and 0.2μg of pSE1UKpro1-2 all obtained above were dissolved in 30μℓ of solution containing 66mM Tris-HCℓ (pH 7.6), 6.6mM MgCℓ₂, 10mM DTT and 1mM ATP, followed by addition of 150 units of T4 DNA ligase to carry out ligation reaction for 18 hours at 4°C. *Escherichia coli* strain MM294 was transformed by using the reaction solution to obtain an ampicillin-resistance strain. Plasmid DNA was recovered from this transformed strain and then subjected to restriction enzyme analysis to obtain pCUO (see Fig. 5).

This plasmid contained bovine αS1 casein chromosomal gene in the range from the site approx. 3.6 kb upstream of the 5'-end to just before exon 2 and, just behind the same, human prourokinase in the whole range from 5'-non-coding region (76 bp) to 3'-non-coding region (76 bp). Furthermore, a splicing signal and a poly A signal of rabbit β-globin gene and a poly A signal of SV40 were contained downstream from the same. Accordingly, it could be expected that this plasmid had a high expression efficiency in the mammary gland.

Example 5

Construction of Human Prourokinase-expressing Vectors pCUW and pCUT Carrying Signal Sequences of Bovine αS1 Casein (See Fig. 6)

5μg of pCUO obtained in Example 4 was dissolved in 40μℓ of solution containing 10mM Tris-HCℓ (pH 7.5) and 7mM MgCℓ₂, followed by addition of 10 units of *Kpn* 1 to carry out digestion reaction for 2 hours at 37°C. Then, NaCℓ was added to the reaction solution to give a final concentration of 50mM, followed by addition of 10 units of *Hind* III to carry out digestion reaction for 2 hours at 37°C. The reaction solution was subjected to agarose gel electrophoresis to isolate and recover a fragment of approx. 11.3 kb. Whereby, approx. 1.5μg of the fragment was obtained.

Then, 10μg of pSE1UKpro1-2 obtained in Example 3 was dissolved in 100μℓ of solution containing 10mM Tris-HCℓ (pH 7.5), 7mM MgCℓ₂ and 50mM NaCℓ, followed by addition of 15 units of *Taq* I to carry out digestion reaction for 2 hours at 65°C. Subsequently, 15 units of *Pst* I was added to the reaction solution to carry out digestion reaction for 2 hours at 37°C. The reaction solution was phenol extracted, ethanol precipitated and then subjected to agarose gel electrophoresis to isolate and recover a fragment of 280 bp. Whereby, approx. 0.05μg of the fragment was obtained.

Then, 5μg of pSE1UKpro1-2 was dissolved in 40μℓ of solution containing 10mM Tris-HCℓ (pH 7.5) and 7mM MgCℓ₂, similarly, followed by addition of 10 units of *Kpn* I to carry out digestion reaction for 2 hours at 37°C. To the reaction solution, were added NaCℓ to give a final concentration of 100mM and then 10 units of *Pst* I to carry out digestion reaction for 2 hours at 37°C. The reaction solution was subjected to agarose gel electrophoresis to isolate and recover a fragment of approx. 1 kb. Whereby, approx. 0.3μg of the fragment was obtained.

Separately, synthetic DNA linkers containing a signal sequence of αS1 casein and a sequence arround N-terminal of mature human prourokinase were prepared. The sequences of these linkers are shown in next page.

Linker 1

Bovine αS1 Casein Signal Peptide

Initiation→

Hind III

```
5'- AGCTTGACAACC ATGAAACTTCTTATCCTCACGTGTCTTGTGGCTGTTGCTCTTGCC
3'-     ACTGTTGG TACTTTGAAGAATAGGAGTGCACAGAACACCGACAACGAGAACGG
                 MetLysLeuLeuIleLeuThrCysLeuValAlaValAlaLeuAla
```

Mature Human Prourokinase→

Taq I

```
AGCAATGAACTTCATCAAGTTCCAT        -3'
TCGTTACTTGAAGTAGTTCAAGGTAGC      -5'
SerAsnGluLeuHisGlnValProSer
```

Linker 2

Bovine αS1 Casein Signal Peptide

Initiation→

Hind III

```
5'- AGCTTGACAACC ATGAAACTTCTTATCCTCACGTGTCTTGTGGCTGTTGCTCTTGCC AGG
3'-     ACTGTTGG TACTTTGAAGAATAGGAGTGCACAGAACACCGACAACGAGAACGG TCC
                 MetLysLeuLeuIleLeuThrCysLeuValAlaValAlaLeuAlaArg
```

Mature Human Prourokinase→

Taq I

```
AGCAATGAACTTCATCAAGTTCCAT        -3'
TCGTTACTTGAAGTAGTTCAAGGTAGC      -5'
SerAsnGluLeuHisGlnValProSer
```

In Linker 1, the signal sequence of αS1 casein and mature human prourokinase are linked. In Linker 2, one amino acid (Arg) is added to the linkage site. The linkers are used upon phosphorylation as same as those of Examples 2 and 4.

0.1μg of Hind III-Kpn I fragment of the pCUO, 0.02μg of Taq I-Pst I fragment and 0.2μg of Pst I-Kpn I fragment of the pSE1UKpro1-2 and 0.01μg of Linker 1 (in case of pCUW) or 0.01μg of Linker 2 (in case of pCUT) were dissolved in 30μl of solution containing 66mM Tris-HCl (pH 7.6), 6.6mM MgCl$_2$, 10mM DTT and 1mM ATP, followed by addition of approx. 150 units of T4 DNA ligase to carry out ligation reaction for 24 hours at 4°C. Then, Escherichia coli strain MM294 was transformed by using the reaction solution to obtain ampicillin-resistance strains. Plasmid DNAs were recovered from these strains and then subjected to restriction enzyme analysis to obtain pCUW and pCUT. The construction process of these plasmids were shown in Fig. 6.

Example 6

Preparation of Transgenic Mice Carrying Fused Gene of Bovine αS1 Casein Chromosomal Gene and Human Prourokinase cDNA

With respect to a method for preparing a transgenic mouse, the present invention followed "How to prepare a transgenic mouse" in *A Laboratory Manual of Embryological Engineering* [Tatsuji Nomura (supervised), Motoya Katsuki (ed.), Kodansha Scientific (1987)]. DNA to be used here was prepared by cutting pCUO and pCUW with *Xho* I and *Cla* I and subsequently obtaining approx. 8.2-kb fragments containing a part of bovine $\alpha$S1 casein gene, human prourokinase cDNA, a splicing signal and a poly A signal of rabbit $\beta$-globin gene and a poly A signal of SV40. These DNA fragments were subjected to injection into pronucleus. The approx. 8.2-kb fragments were dissolved in a Dulbecco's PBS solution (manufactured by GIBCO Inc.) to give a concentration of approx. 20ng/$\mu\ell$, 1 to 2p$\ell$ of which was then injected into the pro-nucleus of a fertilized egg with a microcapillary pipet. After the injection, the fertilized egg was transferred to the oviduct of a recipient mouse with a micro glass capillary. After approx. 3 weeks, offsprings developed from the fertilized eggs were obtained.

The strains of mice used for supplying fertilized eggs were F1 crossbreeds of C57BL/6 $\times$ Balb/c (hereinafter abbreviated to B6C), which were mated to obtain fertilized eggs. A recipient for a fertilized egg, a foster mother and a male mouse for preparing a foster mother used were all ICR (MCH) (manufactured by Japan Clea Co., Ltd.).

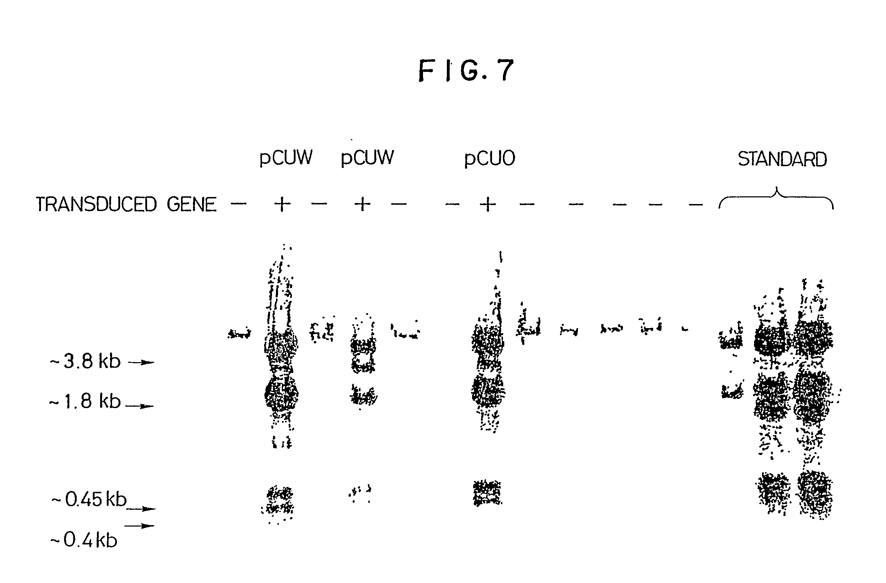

A part of tail of a bred mouse (aged around 4 weeks after birth) was cut, from which high molecular weight DNA was extracted. The DNA was subjected to Southern blot hy bridization by using a *Xho* I-*Cla* I fragment of the aforementioned pCUW as a probe to thereby detect a transgenic mouse carrying the DNA on its chromosome. As the result, 3 transgenic mice with respect to pCUW and 10 transgenic mice with respect to pCUO were obtained. One example of the results of this Southern blot hybridization was shown in Fig. 7.

In Fig. 7, 3.8-kb bands correspond to fragments from *Bgl* II on the upstream of a casein promoter to *Bgl* II existing in a proUK cringle, 1.8-kb bands correspond to fragments from *Bgl* II in the proUK cringle to *Bgl* II just behind a splicing signal of rabbit $\beta$-globin, 450-bp bands correspond to fragments containing a poly A signal of rabbit $\beta$-globin, and 400-bp bands correspond to fragments between two *Bgl* II sites existing upstream of the 5′-end of a casein promoter. The standard indicates DNA fragments of CUW digested with *Bgl* II.

Example 7

Production of Human Prourokinase in Milk by Transgenic Mice Carrying Sequences of pCUO and pCUW on their Cromosome

Female transgenic mice obtained in Example 6 were mated with male B6C mice to breed offsprings. In the nursing period (approx. 2nd week after delivery), milk was collected from the female mice (approx. 0.3m$\ell$). After centrifuging the milk (15,000 rpm, 5 min., 4°C, a high-speed microcentrifuge manufactured by Tomy Co., Ltd.), skim milk of the lower layer was separated. The activity of human prourokinase in the skim milk was measured by fibrin plate assay [Granelli-Peperno and Reich, *J. Exp. Med.*, vol. 148, p. 223 (1978)]. As the result, approx. 15 units/m$\ell$ (approx. 0.3$\mu$g/m$\ell$) of activity was detected in two individuals, CUO 7-6$\ell$ and CUW 13-1$\ell$ and, thus, it was confirmed that human prourokinase was secreted and produced in milk of the transgenic mice.

On the other hand, CUO 7-6$\ell$ and CUW 13-1$\ell$ were respectively mated with normal B6C mice to obtain offsprings. As the result, the transduced genes were found in 3 offsprings out of 6 of the CUO 7-6$\ell$ and in 5 offsprings out of 10 of the CUW 13-1$\ell$, so that it was confirmed that the transmission pattern was normal.

Consequently, it was proved that these two kinds of transduced genes were stably maintained in transgenic mice and transmitted to their offsprings.

Example 8

Purification of Human Prourokinase from Milk of Transgenic Mouse

Approx. 1m$\ell$ of milk of CUW 13-1$\ell$ obtained in Example 7 (containing approx. 15 units in terms of urokinase activity) was diluted 5-fold with a dilution buffer [a phosphate buf fer (pH 7.5) containing 0.01% Tween 80, 0.05% NaN₃, 100mM NaCℓ and 200mM arginine]. After centrifuging the resulting solution (15,000 rpm, 5 min., 4°C), approx. 4.5mℓ of skim milk of the lower layer was recovered. The skim milk was adjusted to pH 4.5 with 0.1M HCℓ to precipitate casein, followed by centrifugation (15,000 rpm, 5 min., 4°C). Then, approx. 4.5mℓ of supernatant was recovered. After adjusting the pH to 7.5 with NaOH, the supernatant was dialyzed against 1,000-fold volume of column buffer (the same as the aforementioned dilution buffer except containing no arginine) for 18 hours at 4°C. The dialyzed sample was passed through a column packed with 1-mℓ anti-urokinase monoclonal antibody column [prepared by bonding anti-urokinase monoclonal antibodies to Pharmacia CNBr-active Sepharose 4B (manufactured by Pharmacia Japan Co., Ltd.)] equilibrated with the aforementioned column buffer.

Then, the 3 bed-volume of the aforementioned column buffer was passed, followed by elution with 4mℓ of 2mM phosphate-50mM citrate buffer (pH 4.0) containing 0.01% Tween 80, 0.05% NaN₃, 100mM NaCℓ and 200mM arginine to fraction by 0.5mℓ portions. The eluate was immediately adjusted to pH 7.5 with phosphoric acid and then dialyzed against the aforementioned dilution buffer for 18 hours at 4°C. The dialyzed sample was assayed for urokinase activity according to fibrin plate assay as same as in Example 7 to detect 5 units of urokinase activity in total. Whereby, the purification and the recovery of human prourokinase were confirmed.

Referential Example 1

Construction of Plasmid ptPA7 Carrying Human t-PAcDNA

(1) Preparation of Poly(A) RNA from Detroit 562 Cell

RNA carrying poly(A) was prepared from human pharynx cancer cell strain Detroit 562 according to guanidine thiocyanate-lithium chloride method [Cathala et al, DNA, vol. 2, p. 329 (1983)] in the following manner.

The human pharynx cancer Detroit 562 cells [W. D. Peterson, Jr. et al, Proc. Soc. Exp. Biol. Med., vol. 136, p. 1187 (1971)] was grown in a tissue culture flask (150cm³, manufactured by Corning Corp.) by using 50mℓ of MEM medium (manufactured by Nissui Seiyaku K.K.) containing 10% fetal calf serum, 1/100 volume of 100× inessential amino acid solution (manufactured by Flow Laboratories, Inc.), 1mM sodium pyruvate and 0.1% lactalbumin hydrate (manufactured by GIBCO Oriental, Inc.). After culturing to the confluence at 37°C, the cells were washed with PBS. Then, 100ng/mℓ of phorbol myristate acetate (PMA) and, subsequently, 30mℓ of the above medium excluding fetal calf serum was added to the washed cells, followed by culturing for 24 hours at 37°C. Then, the cultured cells were treated with 10mℓ of solution containing 0.05% trypsin and 0.02% EDTA to obtain a cell suspension. From the above 6 tissue culture flasks, 1×10⁸ cells were obtained in total. Then, cells were collected form the cell suspension by centrigugation (1,100×g, 4°C, 10 min.), washed with 80mℓ of phosphate buffer. The washed cells were solubilized in 10mℓ of solution containing 5M guanidine thiocyanate, 10mM EDTA, 50mM Tris-HCℓ (pH 7) and 8%(V/V) 2-mercaptoethanol by using a vortex mixer. The solubilized cells were transferred to a centrifugal tube, stirred after adding thereto 80mℓ of 4M LiCℓ solution and then allowed to stand for 20 hours at 4°C. After centrifuging the solution in a Hitachi RPR10 rotor (9,000 rpm, 90 min.), RNA was recovered as a precipitate. The RNA precipitate was suspended in 50mℓ of solution containing 4M urea and 2M lithium chloride. After centrifuging the suspension (9,000 rpm, 60 min.), RNA was recovered again as a precipitate. The RNA precipitate was dissolved in 10mℓof solution containing 0.1% sodium lauryl sulfate, 1mM EDTA and 10mm Tris-HCℓ (pH 7.5) and then extracted with phenol-chloroform. The extract was ethanol precipitated to recover RNA. Approx. 2.5mg of the obtained RNA was dissolved in 1mℓ of solution containing 10mM Tris-HCℓ (pH 8.0) and 1mM EDTA. After incubating this solution for 5 minutes at 65°C, 0.1mℓ of 5M NaCℓ was added thereto. The mixture was subjected to chromatography using an oligo(dT)-cellulose column (column volume, 0.5mℓ; manufactured by P-L Biochemicals, Inc.). The adsorbed mRNA carrying poly(A) was eluted with a solution containing 10mM Tris-HCℓ (pH 7.5) and 1mM EDTA to obtain approx. 90μg of mRNA carrying poly(A).

(2) Synthesis of cDNA and Insertion of the cDNA into a Vector

13

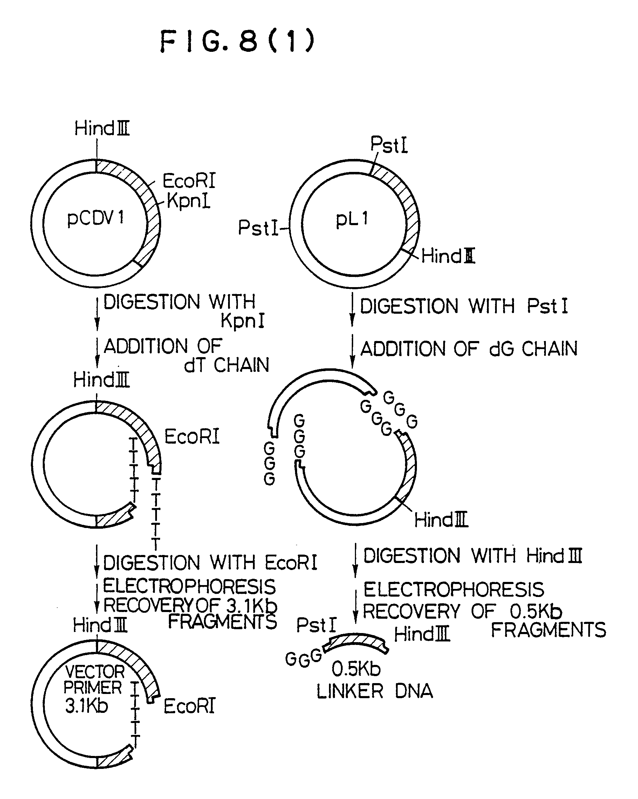

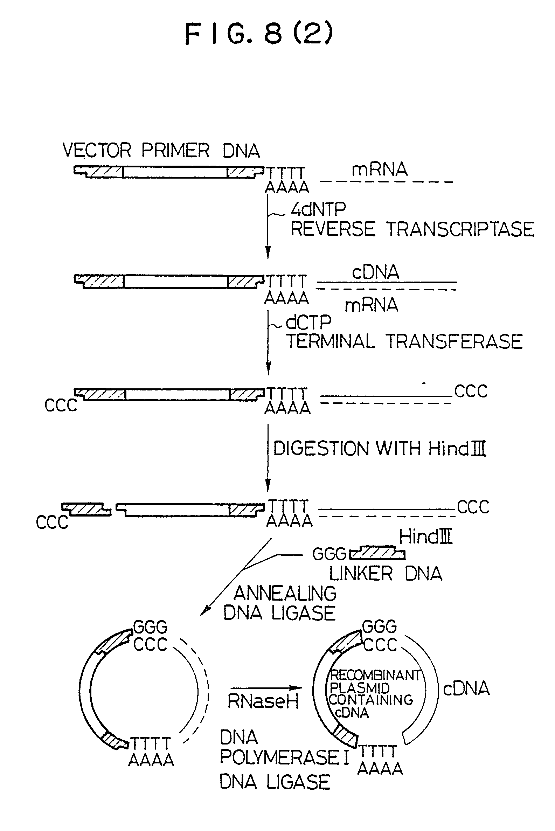

According to Okayama-Berg method [*Mol. Cell. Biol.*, vol. 2, p. 161 (1982)], the synthesis of cDNA and the construction of a recombinant plasmid in which the cDNA was integrated were carried out. The schematic process thereof is shown in Fig. 8.

400μg of pCDV1 [Okayama and Berg, *Mol. Cell. Biol.*, vol. 3, p. 280 (1983)] was added to 300μg of solution containing 10mM Tris-HCl (pH 7.5), 6mM MgCl$_2$ and 10mM NaCl, followed by addition of 500 units of *Kpn* I to carry out reaction for 6 hours at 37°C. Whereby, the pCDV was cut at its *Kpn* I site. After the extraction with phenol-chloroform, DNA was recovered by ethanol precipitation. Approx. 200μg of the DNA cut with *Kpn* I was added to 200μl of solution prepared by adding dTTP to a buffer (hereinafter abbreviated to "TdT buffer") containing 40mM sodium cacodylate, 30mM Tris-HCl (pH 6.8), 1mM CaCl$_2$ and 0.1mM dithiothreitol (hereinafter abbreviated to "DTT") so as to give a final dTTP concentration of 0.25mM, followed by addition of 81 units of terminal deoxynucleotidyl transferase (hereinafter abbreviated to "TdT"; manufactured by P-L Biochemicals, Inc.) to carry out reaction for 11 minutes at 37°C. In this reaction, approx. 67 poly(dT) residues were added to the 3'-end of the *Kpn* I-site of pCDV1. From the solution, 100μg of poly(dT)added pCDV1 DNA was recovered by phenol-chloroform extraction and ethanol precipitation. The DNA was added to 150μl of buffer containing 10mM Tris-HCl (pH 7.5), 6mM MgCl$_2$ and 100mM NaCl, followed by addition of 360 units of *Eco* RI to carry out reaction for 2 hours at 37°C. After treating the reaction solution according to LGT method, approx. a 3.1-kb DNA fragment was recovered and approx. 60μg of poly(dT)-added pCDV1 was obtained. The DNA was dissolved in 500μl of solution containing 10mM Tris-HCl (pH 8.0) and 1mM EDTA, incubated for 5 minutes at 65°C and ice-cooled. To the resulting solution, was added 50μl of 5M NaCl. The mixture was subjected to chromatography using an oligo(dA)-cellulose column (manufactured by Colaborative Research, Inc.). Poly(dT) residues having enough length were adsorbed onto the column. The adsorbed poly(dT) was eluted with a solution containing 10mM Tris-HCl (pH 8.0) and 1mM EDTA to obtain 27μg of poly(dT)-added pCDV1 (hereinafter abbreviated to "vector primer").

Then, the preparation of linker DNA was carried out.

Approx. 14μg of pL1 [Okayama & Berg, *Mol. Cell. Biol.*, vol. 3, p. 280 (1983)] was added to 200μl of buffer contain ing 10mM Tris-HCl (pH 7.5), 6mM MgCl$_2$ and 50mM NaCl, followed by addition of 50 units of *Pst* I to carry out reaction for 4 hours at 37°C. In this reaction, pL1DNA was cut at its *Pst* I site. The reaction mixture was phenol-chloroform extracted and then ethanol precipitated to recover approx. 13μg of pL1DNA cut with *Pst* I. Approx. 13μg of the DNA was added to 50μl of solution prepared by adding dGTP to a TdT buffer to give a final dGTP concentration of 0.25mM, followed by addition of 54 units of TdT (manufactured by P-L Biochemicals, Inc.). The mixture was incubated for 13 minutes at 37°C to add approx. 14 (dG) residues to the 3'-end of *Pst* I-cut site of the pL1. After the phenol-chloroform extraction, DNA was recovered by ethanol precipitation. The DNA was added to 100μl of buffer containing 10mM Tris-HCl (pH 7.5), 6mM MgCl$_2$ and 60mM NaCl, followed by addition of 80 units of *Hind* III. The mixture was incubated for 3 hours at 37°C to cut pL1DNA at its *Hind* III site. The reaction mixture was fractioned by agarose gel electrophoresis, followed by recovering as approx. 0.5-kb DNA fragment by DEAE paper method [Dretzen et al, *Anal. Biochem.*, vol. 112, p. 295 (1981)]. Whereby, oligo (dG) tail-added linker DNA (hereinafter abbreviated to merely "linker DNA") was obtained.

Approx. 4μg of poly(A) RNA and approx. 1.4μg of vector primer obtained as above, were dissolved in 22.3μl of solution containing 50mM Tris-HCl (pH 8.3), 8mM MgCl$_2$, 30mM KCl, 0.3mM DTT, 2mM dNTP (dATP, dTTP, dGTP and dCTP) and 10 units of ribonuclease inhibitor (manufactured by P-L Biochemicals, Inc.), followed by addition of 10 units of reverse transcriptase (manufactured by Seikagaku Kogyo K.K.). The mixture was incubated for 90 minutes at 41°C to synthesize DNA complementary to mRNA. The reaction mixture was phenol-chloroform extracted and then ethanol precipitated to recover RNA-DNA double strand-added vector primer DNA. The DNA was dissolved in 20μl of TdT buffer containing 66μM dCTP and 0.2μg of poly(A), followed by addition of 14 units of TdT (manufactured by P-L Biochemicals, Inc.). The mixture was incubated for 2 minutes at 37°C to add 20 (dC) residues to the 3'-end of cDNA. The reaction mixture was phenol-chloroform extracted and then ethanol precipitated to recover (dC) tail-added cDNA-vector primer DNA. The DNA was dissolved in 400μl of solution containing 10mM Tris-HCl (pH 7.5), 6mM MgCl$_2$ and 60mM NaCl, followed by addition of *Hind* III. The mixture was incubated for 2 hours at 37°C to cut the DNA at its *Hind* III site. The reaction mixture was phenol-chloroform extracted and then ethanol precipitated to obtain 0.5 picomole of (dC) tail-added cDNA-vector primer DNA. 0.2 picomole of the DNA and 0.4 picomole of the aforementioned linker DNA were dissolved in 100μl of solution containing 10mM Tris-HCl (pH 7.5), 0.1M NaCl and 1mM EDTA, followed by incubation at 65°C, 42°C and 0°C in order respectively for 10, 25 and 30 minutes. The reaction solution was adjusted to give a total volume of 1,000μl by adding the composition of 20mM Tris-HCl (pH 7.5), 4mM MgCl$_2$, 10mM (NH$_4$)$_2$SO$_4$, 0.1M KCl and 0.1mM β-NAD. Then, 25 units of *Escherichia coli* DNA ligase (manufactured by New England BioLabs, Inc.) was added to the reaction solution and incubated for 18 hours at 11°C. The reaction solution was adjusted by adding dNTP and β-NAD so that the concentrations thereof might reach 40μm and 0.15mM respectively, followed by addition of 20 units of *Escherichia coli* DNA polymerase I (manufactured by P-L Biochemicals, Inc.) and 10 units of *Escherichia coli* ribonuclease H (manufactured by P-L Biochemicals, Inc.). The mixture was incubated at 12°C and 25°C in order respctively for 1 hour. In the above reaction, the circularization of recombinant DNA containing cDNA and the substitution of RNA part of RNA-DNA double strand by DNA were attained to form a recombinant plasmid carrying complete double-stranded DNA.

(3) Selection of Recombinant DNA containing Human t-PA•cDNA

Emplying colony hybridization, t-PA•cDNA was selected as a clone associating with a probe prepared by labelling a synthetic DNA having a base sequence of
5′-ATGGATGCAATGAAGAGAGGGCTCTGCTGT-3′
coincident with a part of the base sequences of the t-PA signal peptide region of human t-PA cDNA [Pennica et al, *Nature*, vol. 301, p. 214 (1983)] with P$^{32}$ in the following manner.

Firstly, *Escherichia coli* strain C600SF8 [Cameron, *Proc. Natl. Acad. Sci.*, U.S.A., vol. 72, p. 3416 (1975)] was transformed according to Hanahan's method [Hanahan, *J. Mol. Biol.*, vol. 166, p. 557 (1983)] with a recombinant plasmid obtained in (2). The obtained approx. 10,000 colonies were fixed on a nitrocellulose filter according to the Hanahan-Meselson Method [Hanahan & Meselson, *Method In Enzymology*, vol. 100, p. 333 (1983)]. Then, the filter was prehybridized in a solution containing 6× NET [1× NET = 150mM NaCl, 15mM Tris-HCl (pH 7.5) and 1mM EDTA], 10× Denhardt's solution and 100μg/ml of fragmented *Escherichia coli* chromosomal DNA for 4 hours or more at 65°C. To this prehybridization solution, was added the above $^{32}$P-labelled probe to associate with DNA on the filter (65°C, 16 hours or more). Then, the filter was washed twice with 6× SSC (1× SSC = 150mM NaCl and 15mM sodium citrate) at room temperature for 5 minutes each and then with a solution of 65°C containing 2× SSC and 0.1% SDS for 30 minutes. Furthermore, the filter was washed with a solution of 65°C containing 2× SSC and 0.1% SDS for 15 minutes and then twice with 6× SSC at room temperature (5 minutes each). After air-drying, the filter was autoradiographed to identify one positive clone. The base sequence of plasmid ptPA7 cDNA carried by this positive clone was determnined by dideoxy sequencing method using an M13 phage. As the result, it was proved that the ptPA7 cDNA coded for t-PA which was completely coincident with the t-PA amino acid sequence reported by Pennica [Pennica et al, *Nature*, vol. 301, p. 214 (1983)], where the 95th aspartic acid codon (GAC) and the 512th threonine codon (ACA) were found to be converted to GAT and ACC respectively.

This strain was deposited with the Fermentation Research Institute as *Escherichia coli* EtPA7 (FERM BP-1467).

Referential Example 2

Construction of Plasmid pUK1 Carrying Human pro-UK cDNA

The cDNA library of Detroit 562 cell prepared in Referential Example 1 was screened according to colony hybridization method to isolate human pro-UK cDNA clones. That is, *Escherichia coli* strain C600SF8 [Cameron, *Proc. Natl. Acad. Sci.*, U.S.A., vol. 72, p. 3416 (1975)] was transformed by using a recombinant plasmid obtained in Referential Example 1 according to Hanahan's method [Hanahan, *J. Mol. Biol.*, vol. 166, p. 557 (1983)] firstly. The obtained approx. 30,000 colonies were fixed on a nitrocellulose filter according to the Hanahan-Meselson method [Hanahan & Meselson, *Methods in Enzymology*, vol. 100, p. 333 (1983)]. Then, the filter was prehybridized in a solution containing 6× NET, 10× Denhardt's solution and 100μg/ml of fragmented *Escherichia coli* chromosomal DNA at 65°C for 4 hours or more.

Then, a probe prepared by labelling a synthetic DNA carrying 41 bases,
5′-GGGAATGGTCACTTTTACCGAGGAAAGGCCAGCACTGACAC-3′
(corresponding to the underlined base sequence in Table 5 with regard to the human pro-UK cDNA isolated by the present inventors), coinsident with a part of the base sequence of the cringle region of human pro-UK cDNA [Holmes et al, *Bio/technology*, vol. 3, p. 923 (1985)] with $^{32}$P was added to the above prehybridization solution to thereby associate with DNA on the filter (65°C, 16 hours or more). The filter was washed twice with 6× SSC (at room temperature, for 5 minutes each) and then with a solution of 57°C containing 1× SSC and 0.1% SDS for 30 minutes. Furthermore, the filter was washed first with a solution of 57°C containing 1× SSC and 0.1% SDS for 15minutes and then twice with 6× SSC (at room temperature, for 5 minutes each). The filter was air-dried and then autoradiographed to identify one positive clone. The base sequence of the plasmid pUK1 cDNA carried by this positive clone was determined according to dideoxy sequencing method using an M13 phage (Table 1). As the result, it became clear that the pUK1 cDNA coded for the coding region and the 3'-non-coding region of pro-UK downstream of the 41st Cys-residue of the pro-UK in case of following the numbering of amino acid residues of pro-UK in Table 1. Although the amino acid sequences coded by the pUK1 cDNA were coincident with those reported by Holmes et al [Holmes et al, *Bio/technology*, vol. 3, p. 923 (1985)], each the 3rd base of the following four amino acid codons was differed:

The 254th amino acid Asn: AAC → AAT
The 340th amino acid Leu: CTA → CTG
The 345th amino acid Pro: CCC → CCA
The 346th amino acid Gln: CAA → CAG

This strain was deposited with the Fermentation Research Institute as *Escherichia coli* EUK1 (FERM BP-1463).

EP 0 390 592 A2

Table 1 - 1

ATGAGAGCCCTGCTGGCGCGCCTGCTTCTCTGCGTCCTGGTCGTGAGCGACTCCAAAGGCAGCAATGAACTTCAT
MetArgAlaLeuLeuAlaArgLeuLeuLeuCysValLeuValValSerAspSerLysGlySerAsnGluLeuHis

CAAGTTCCATCGAACTGTGACTGTCTAAATGGAGGAACATGTGTGTCCAACAAGTACTTCTCCAACATTCACTGG
GlnValProSerAsnCysAspCysLeuAsnGlyGlyThrCysValSerAsnLysTyrPheSerAsnIleHisTrp

TGCAACTGCCCAAAGAAATTCGGAGGGCAGCACTGTGAAATAGATAAGTCAAAAACCTGCTATGAGGGGAATGGT
CysAsnCysProLysLysPheGlyGlyGlnHisCysGluIleAspLysSerLysThrCysTyrGluGlyAsnGly

CACTTTTACCGAGGAAAGGCCAGCACTGACACCATGGGCCGGCCCTGCCTGCCCTGGAACTCTGCCACTGTCCTT
HisPheTyrArgGlyLysAlaSerThrAspThrMetGlyArgProCysLeuProTrpAsnSerAlaThrValLeu

CAGCAAACGTACCATGCCCACAGATCTGATGCTCTTCAGCTGGGCCTGGGGAAACATAATTACTGCAGGAACCCA
GlnGlnThrTyrHisAlaHisArgSerAspAlaLeuGlnLeuGlyLeuGlyLysHisAsnTyrCysArgAsnPro

GACAACCGGAGGCGACCCTGGTGCTATGTGCAGGTGGGCCTAAAGCCGCTTGTCCAAGAGTGCATGGTGCATGAC
AspAsnArgArgArgProTrpCysTyrValGlnValGlyLeuLysProLeuValGlnGluCysMetValHisAsp

TGCGCAGATGGAAAAAAGCCCTCCTCTCCTCCAGAAGAATTAAAATTTCAGTGTGGCCAAAAGACTCTGAGGCCC
CysAlaAspGlyLysLysProSerSerProProGluGluLeuLysPheGlnCysGlyGlnLysThrLeuArgPro

CGCTTTAAGATTATTGGGGGAGAATTCACCACCATCGAGAACCAGCCCTGGTTTGCGGCCATCTACAGGAGGCAC
ArgPheLysIleIleGlyGlyGluPheThrThrIleGluAsnGlnProTrpPheAlaAlaIleTyrArgArgHis

CGGGGGGGGCTCTGTCACCTACGTGTGTGGAGGCAGCCTCATCAGCCCTTGCTGGGTGATCAGCGCCACACACTGC
ArgGlyGlySerValThrTyrValCysGlyGlySerLeuIleSerProCysTrpValIleSerAlaThrHisCys

TTCATTGATTACCCAAAGAAGGAGGACTACATCGTCTACCTGGGTCGCTCAAGGCTTAACTCCAACACGCAAGGG
PheIleAspTyrProLysLysGluAspTyrIleValTyrLeuGlyArgSerArgLeuAsnSerAsnThrGlnGly

## Table 1 - 2

```
GAGATGAAGTTTGAGGTGGAAAAACCTCATCCTACACAAGGACTACAGCGCTGACACGCTTGCTCACCACAATGAC
GluMetLysPheGluValGluAsnLeuIleLeuHisLysAspTyrSerAlaAspThrLeuAlaHisHisAsnAsp

ATTGCCTTGCTGAAGATCCGTTCCAAGGAGGGGCAGGTGTGCGCAGCCATATACAGACCATCTGCCTG
IleAlaLeuLeuLysIleArgSerLysGluGlyArgCysAlaGlnProSerArgThrIleGlnThrIleCysLeu

CCCTCGATGTATAACGATCCCCAGTTTGGCACAAGCTGTGAGATCACTGGCTTTGGAAAAGAGAATTCTACCGAC
ProSerMetTyrAsnAspProGlnPheGlyThrSerCysGluIleThrGlyPheGlyLysGluAsnSerThrAsp

TATCTCTATCCGGAGCAGCTGAAAATGACTGTTGTGAAGCTGATTTCCCACCGGGAGTGTCAGCAGCCCCACTAC
TyrLeuTyrProGluGlnLeuLysMetThrValValLysLeuIleSerHisArgGluCysGlnGlnProHisTyr

TACGGCTCTGAAGTCACCACCAAAATGTCTGTGTGCTGCTGACCCACAGTGGAAAACAGATTCCTGCCAGGGAGAC
TyrGlySerGluValThrThrLysMetSerValCysCysLeuAlaAlaAlaAspProGlnTrpLysThrAspSerCysGlnGlyAsp

TCAGGGGGACCCCTCGTCTGTTCCCTCCAAGGCCGCATGACTTTGACTGGAATTGTGACTGGGGCCGTGGATGT
SerGlyGlyProLeuValCysSerSerLeuGlnGlyArgMetThrLeuThrGlyIleValSerTrpGlyArgGlyGlyCys

GCCCTGAAGGACAAGCCAGGCGTCTACACGAGAGTCTCACACTTCTTACCCTGGATCCGCAGTCACACCAAGGAA
AlaLeuLysAspLysProGlyValTyrThrArgValSerHisPheLeuProTrpIleArgSerHisThrLysGlu

GAGAATGGCCTGGCCCTCTGA
GluAsnGlyLeuAlaLeu***
```

Referential Exmaple 3

### Construction of Plasmid pUK11 carrying Human pro-UK cDNA

The pro-UK cDNA coded by the plasmid pUK1 obtained in Referential Example 2 did not contain the signal region and the growth factor domain region of pro-UK, so that the cloning of cDNA containing these regions was carried out by taking the following procedures.

Firstly, vector pCCK2 to be used for the cloning of cDNA was constructed in the following manner.

18

(1) Construction of Recombinant plasmid pCCK1

*Escherichia coli* strain H101 carrying rat cerebral cholecystokinin constructed by Kuwano et al [CCK = plasmid pRC19 carrying precursor cDNA; Kuwano et al, *J. Biochem.*, vol. 96, pp. 923~926 (1984)] was cultured. From the cultured microorganism, pRC19 DNA was prepared according to an ordinary method. Approx. $3\mu$g of the obtained pRC19 DNA was dissolved in $30\mu l$ of Y-50 buffer [10mM Tris-HCl (pH 7.5), 7mM $MgCl_2$, 6mM 2-mercaptoethanol, 50mM NaCl], followed by addition of 1 unit of *Pvu* II to carry out digestion reaction for 1 hour at 37°C. By this reaction, DNA was partially digested by *Pvu* II. After the thermal treatment at 65°C for 10 minutes, an approx. 530-bp DNA fragment was purified according to AFT method. On the other hand, approx. $1\mu$g of plasmid DNA pUC19 constructed by Norrander et al [J. Norrander et al, *Gene*, vol. 26, p. 101 (1983); pUC19 plasmid DNA is available from Takara Shuzo Co., Ltd.] was dissolved in $30\mu l$ of Y-O buffer [10mh Tris-HCl (pH 7.5), 7mM $MgCl_2$, 6mM 2-mercaptoethanol] containing 20mM KCl, followed by addition of 16 units of *Sma* I to carry out digestion reaction for 2 hours at 30°C. After the thermal treatment at 65°C for 10 minutes, an approx. 2.7-kb DNA fragment was purified according to AFT method.

Approx. $0.01\mu$g of the pRC19-derived DNA fragment (approx. 530 bp) and approx. $0.05\mu$g of the pUC19-derived DNA fragment (approx. 2.7 kb) obtained as described above were dissolved in $20\mu l$ of T4 ligase buffer, followed by addition of 200 units of T4 DNA ligase to carry out ligation reaction for 18 hours at 4°C.

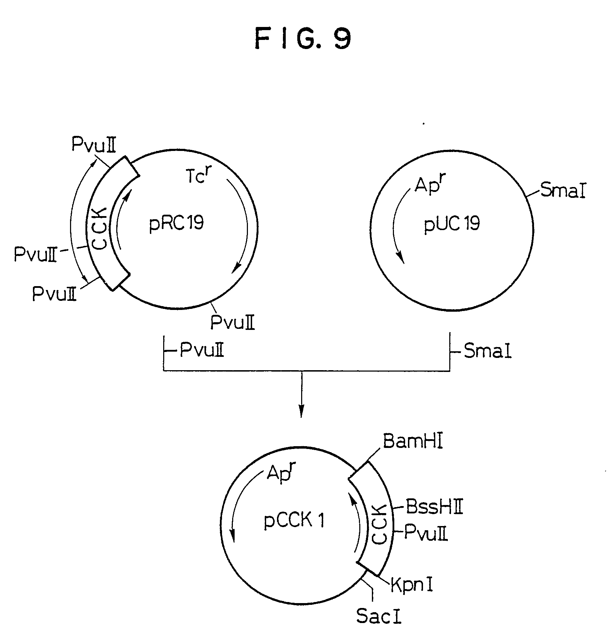

Using the obtained mixture of recombinant plasmids, *Escherichia coli* strain MM294 was transformed to obtain an Ap-resistance strain. From this transformed strain, plasmid DNA pCCK1 was isolated. As a result of structural analysis using restriction enzymes, it was confirmed that this plasmid had the aimed structure (see Fig. 9).

(2) Construction of Recombinant Plasmid pCCK2

Approx. $2\mu$g of pCCK1 plasmid DNA was dissolved in $30\mu l$ of Y-O buffer, followed by addition of 12 units of *Sac* I to carry out digestion reaction for 2 hours at 37°C. Further, $1.5\mu l$ of 2M NaCl and 10 units of *Bam* HI were added to carry out digestion reaction for 2 hours at 37°C. After the thermal treatment at 65°C for 10 minutes, an approx. 0.55-kb DNA fragment was purified according to AFT method.

On the other hand, approx. $2\mu$g of pTrS33 plasmid DNA obtained in Referential Example 4 to be described later was subjected to the same reaction as above. The formed approx. 2.85-kb *Sac* I-*Bam* HI fragment was purified according to AFT method.

Approx. $0.02\mu$g of the pCCK1-derived DNA fragment (approx. 0.55 kb) and approx. $0.1\mu$g of the TrS33-derived DNA fragment (approx. 2.85 kb) obtained as above were dissolved in $20\mu l$ of T4 ligase buffer, followed by addition of 50 units of T4 DNA ligase to carry out ligation reaction for 18 hours at 4°C.

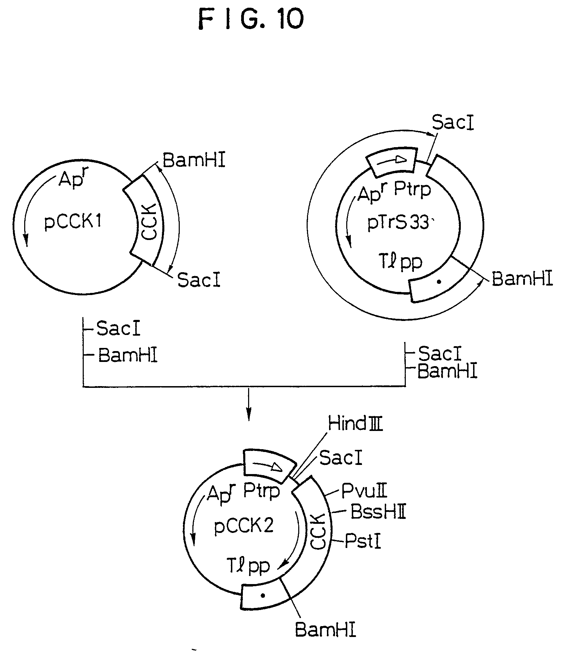

Using the obtained mixture of recombinant plasmids, *Eschericha coli* strain MM294 was transformed to obtain an Ap-resistance strain. From this transformed strain, plasmid DNA pCCK2 was isolated. As a result of structural analysis using ristriction enzymes, it was confirmed that this plasmid had the aimed structure (see Fig. 10).

(3) Isolation of Plasmid pUK11 Carrying Human pro-UK cDNA

Approx. $8\mu$g of poly(A) RNA (mRNA) of the Detroit cell prepared in Referential Example 1 [dissolved in $7\mu l$ of 10mM Tris-HCl (pH 7.5)-0.5mM EDTA] was heated at 65°C for 10 minnutes and then cooled promptly in ice. After adjusting this solution as as to contain 50mM Tris-HCl (pH 8.3), 8mM $MgCl_2$, 30mM KCl, 5mM DTT, 1mM dNTP (dATP, dTTP, dGTP and dCTP), 10 units of ribonuclease inhibitor (manufactured by P-L Biochemicals, Inc.) and $5\mu$g/ml of oligo(dT)$_{12-18}$ (manufactured by Colaborative, Inc.) ($80\mu l$ in total), thus adjusted solution was maintained at 41°C for 15 minutes. Then, 20 units of reverse transcriptase (manufactured by Seikagaku Kogyo K.K.) was added to the solution and maintained at 41°C for 90 minutes to synthesize cDNA complementary to mRNA. The reaction mixture was phenol-chloroform extracted, ethanol precipitated and then dissolved in $4\mu l$ of 0.3M NaOH. The solution was allowed to stand for 15 hours at 37°C to hydrolyze mRNA. After neutralizing the reaction solution by adding $10\mu l$ of 1M Tris-HCl (pH 7.5) and $40\mu l$ of 0.3N HCl, single stranded cDNA was recovered by ethanol precipitation and then dissolved in $28.5\mu l$ of $H_2O$.

This solution was adjusted so as to contain 50mM Tris-HCl (pH 8.3), 8mM $MgCl_2$, 30mM KCl, 5mM

DTT, 1mM dNTP (dATP, dTTP, dGTP and dCTP) and 2.5μg/mℓ synthetic DNA primer CATGAGAGCCCTGCTGG (coincident with a part of base sequence of signal peptide region of human pro-UK)-(40μℓ in total), followed by maintaining at 65°C for 10 minutes and subse quently at 41°C for 30 minutes. Then, 10 units of reverse transcriptase was added to the solution and then maintained at 41°C for 60 minutes to convert the single stranded cDNA top double stranded DNA. The reaction mixture was phenol-chloroform extracted and then ethanol precipitated. After dissolving the precipitate in 30μℓ of Y-O buffer containing 25mM NaCℓ, 25 units of *Bss* HI (manufactured by New England BioLabs, Inc.) was added to carry out digestion reaction for 2 hours at 25°C. Furthermore, 1.25μℓ of 2M NaCℓ and 12 units of *Bam* HII were added to the reaction solution to carry out digestion reaction for 2 hours at 37°C. After heating the reaction solution at 65°C for 10 minutes, an approx. 1.1- to 1.4-kb cDNA fragment was purified according to AFT method.

On the other hand, approx. 2μg of pCCK2 plasmid DNA obtained above was cut with *Bss* HII and *Bam* HI as same as above. Then, as approx. 2.9-kb *Bss* HII-*Bam* HI fragment was purified according to AFT method.

Approx. 0.02μg of the cDNA fragment (approx. 1.1 to 1.4 kb) and approx. 0.05μg of the pCCK2-derived DNA fragment (approx. 2.9 kb) obtained as above were dissolved in 20μℓ of T4 ligase buffer, followed by addition of 200 units of T4 DNA ligase to carry out ligation reaction for 18 hours at 4°C.

Using the obtained mixture of recombinant plasmids, *Escherichia coli* strain C600SF8 was transformed to obtain approx. 25,000 Ap-resistance strains. From among these Ap- resistance strains, approx. 1,000 positive clones associating with the same probe as used for the isolation of pro-UK cDNA according to colony hybridization method. Incidentally, conditions for the hybridization and for the washing of a filter were as same as those in Referential Example 2.

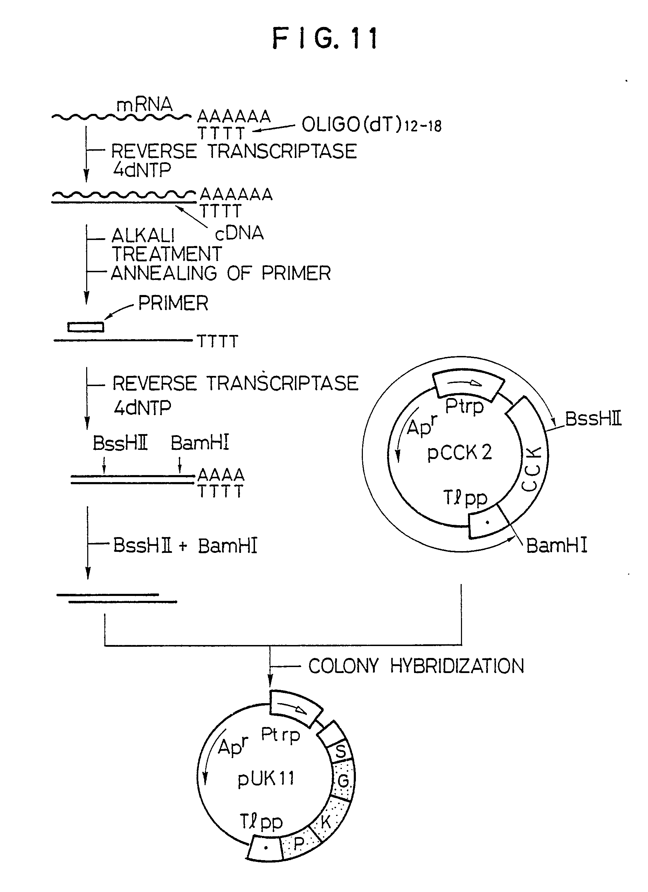

Plasmid pUK11 (see Fig. 11) carried by one positive clone obtained above was isolated and then subjected to the base sequence determination of signal peptide, growth factor domain and cringle regions of pro-UK by dideoxy sequencing method using an M13 phage. As the result, it was confirmed that base sequences thereof were coincident with those reported by Holmes [Holmes et al, *Bio/technology*, vol. 3, p. 923 (1985)].

Referential Example 4

Construction of Recombinant Plasmid pTrS33

Construction of ATG Vector pTrS20

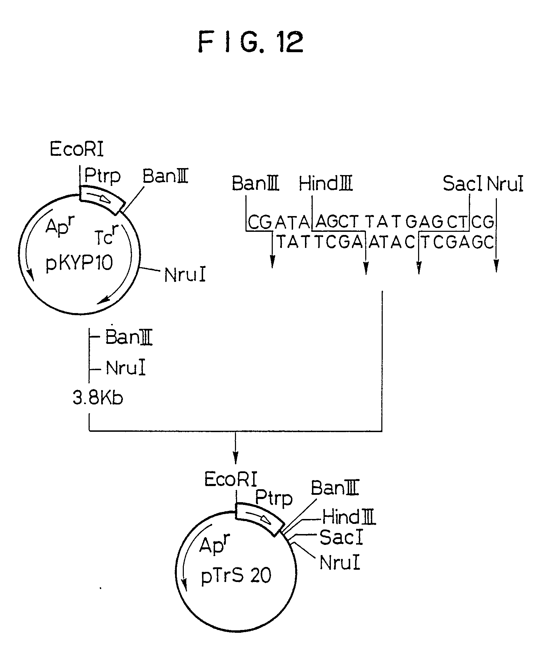

According to the procedure shown in Fig. 12, ATG vector pTrS20 having a distance of 14 bases between the SD sequence and the ATG initiation codon and having *Sac* I site just behind the ATG codon was constructed.

Firstly, 3μg of pKYP10 prepared by the method described in the official gazette of Japanese Patent Application Kokai (Laid-open) No. 110600/1983 was dissolved in 30μℓ of Y-100 buffer [10mM Tris-HCℓ (pH 7.5), 7mM MgCℓ₂, 6mM mercatoetha nol, 100mM NaCℓ], followed by addition of each 6 units of restriction enzymes *Ban* III and *Nru* I (manufactured by New England BioLabs, Inc.) to carry out cutting reaction for 3 hours at 37°. From this reaction solution, approx. 0.5μg of approx. 3.8-kb DNA fragments (*Ban* III-*Nru* I fragments) containing Ptrp was obtained according to LGT method.

On the other hand, the following DNA linker was synthesized according to triester method in order to donate an ATG initiation codon downstream of the Ptrp.

```
        Ban Ⅱ          Hind Ⅲ                    Sac I   Nru I
                                        Met
    5' - |C G A T A|A G C T T  A T G  A G C T|C G|- 3'   (19-mer)

        3' -      |T A T T C G A|A T A C|T C G A G C|- 5'   (17-mer)
```

19-mer and 17-mer synthetic DNAs (10 picomole each) were dissolved in 2-μℓ of solution containing

50mM Tris-HC$\ell$ (pH 7.5), 10mm MgC$\ell_2$, 5mM dithiothreitol, 0.1mM EDTA and 1mM ATP, followed by addition of 3 units of T4 ribonucleotide kinase (manufactured by Takara Shuzo Co., Ltd.) to carry out phosphorylation reaction for 60 minutes at 37°C.

Then, 0.1μg of the pKYP10-derived *Ban* III-*Nru* I fragments (approx. 3.8 kb) obtained above and approx. 0.5 picomole of the above DNA linkers were dissolved in 20μ$\ell$ of T4 ligase buffer, followed by addition of 2 units of T4 DNA ligase to carry out ligation reaction for 18 hours at 4°C.

Using the obtained mixture of recombinant plasmids, *Escherichia coli* strain HB101 [Boliver et al, *Gene*, vol. 2, p. 75 (1977)] was transformed to obtain a colony of Ap$^r$. From the culture of this colony, plasmid DNA was recovered. The structure of the obtained plasmid was confirmed by agarose gel electrophoresis after cutting the plasmid with *Eco* I, *Ban* III, *Hind* III, *Sac* I and *Nru* I. This plasmid was named pTrS2O (Fig. 12). As a result of by dideoxy sequencing using an M13 phage, it was confirmed that the pTrS20 had the following base sequence near the *Ban* III site and the *Hind* III site thereof.

```
                      Ban I   Hind I        Sac I   Nru I
    SD Sequence          |       |      Met     |       |
        AAGG GTAT | CGATA | AGCTT ATG AGCT | CG | CGA
                       ↓        ↓        ↓          ↓
```

(2) Construction of pTrS33

Approx. 3μg of pTrS20 plasmid DNA obtained above was dissolved in 30μ$\ell$ of Y-O buffer, followed by addition of 12 units of *Sac* I to carry out digestion reaction for 2 hours at 37°C. Furthermore, 1.5μ$\ell$ of 2M NaC$\ell$ and 10 units of *Pst* I were added to the reaction solution to carry out digestion reaction for 2 hours at 37°C. After the thermal treatment at 65°C for 10 minutes, an approx. 1.15-kb DNA fragment was purified by AFT method.

On the other hand, 2μg of pKYP26 [*Escherichia coli* con taining pKYP 26 was deposited with the Fermentation Research Institute as *Escherichia coli* IKYP26 (FERM BP-863)] prepared according to a method described in the official gazette of Japanese Patent Application Kokai (Laid-open) No. 48699/1987 was dissolved in 30μ$\ell$ of Y-100 buffer, followed by addition of 8 units of *Pst* I and *Bam* HI to carry out digestion reaction for 2 hours at 37°C. After the thermal treatment at 65°C for 10 minutes, an approx. 1.7-kb DNA fragment was purified by AFT method.

In the meantime, 2μg of M13mp18RF DNA (J. Norrander et al, *Gene*, vol. 26, p. 101 (1983); the M13mp18RF DNA was obtained from Takara Shuzo Co., Ltd.] was dissolved in 30μ$\ell$ of Y-0 buffer, followed by addition of 10 units of *Sac* I to carry out digestion reaction for 2 hours at 37°C. Furthermore, 1.5μ$\ell$ of 1M NaC$\ell$ and 10 units of *Cla* I were added to the reaction solution to carry out digestion reaction for 2 hours at 37°C. After the thermal treatment at 65°C for 10 minutes, an approx. 0.65-kb DNA fragment was purified by AFT method.

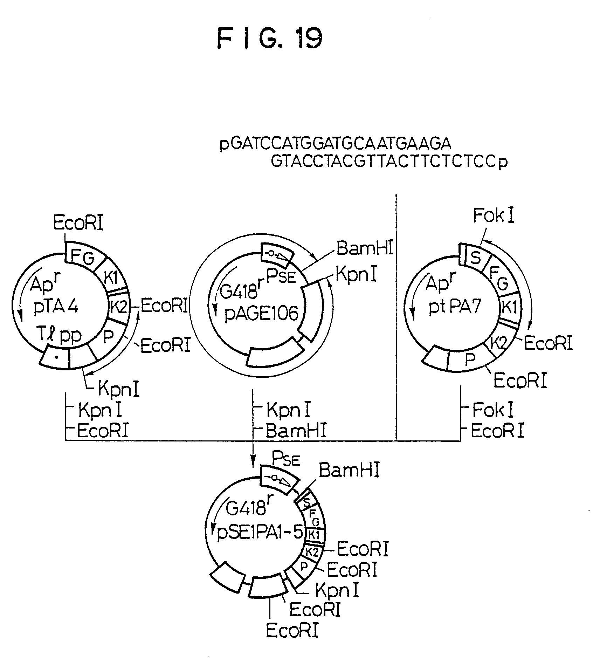

Separately, two kinds of synthetic DNA (43 bases and 45 bases) shown in Fig. 19,

```
                                               (43 bases)
  5'-CGATAAGCTTATGATATCCAACGTCGACGACGGCGTCGAACCATGGCCG-3'
     3'-TATTCGAATACTATAGGTTGCAGCTGCTGCCGCAGCTTGGTACCGGCCTAG-5'
                                               (45 bases),
```

were synthesized by using a 380A-DNA synthesizer (manufac tured by Applied Biosystems, Inc.), which are then 5′-phosphorylated individually in the same manner as described above.

Approx. 0.1μg of the pTrS20-derived DNA fragment (approx. 1.15 kb), approx. 0.1μg of the pKYP26-derived DNA fragment (approx. 1.7 kb) and approx. 0,05μg of the M13mp18-derived DNA frgment (approx. 0.65 kb) obtained as described above and each 1 picomole of two kinds of 5′-phosphorylated synthetic DNAs obtained above were dissolved in 20μl of T4 ligase buffer, followed by addition of 50 units of T4 DNA ligase to carry out ligation reaction for 18 hours at 4°C.

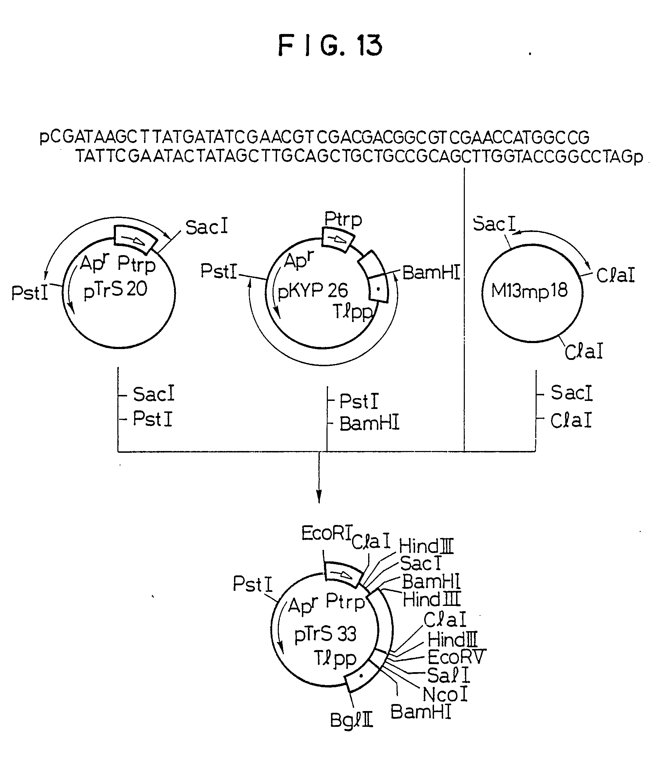

Using thus obtained mixture of recombinant plasmids, *Escherichia coli* strain MM294 was transformed to obtain an Ap-resistance strain. From this transformed strain, plasmid pTrS33 was isolated. It was confirmed that this plasmid had the aimed structure as the result of the structural analysis by restriction enzyme digestion and the dideoxy sequencing using an M13 phage (see Fig. 13).

Referential Example 5

Construction of Recombinant Plasmid pTerm2

Approx. 2μg of pKYP26 plasmid [see the official gazette of Japanese Patent Application Kokai (Laid-open) No. 48699/1987] was dissolved in 3oμl of solution containing 10mM Tris-HCℓ (pH 8.0), 75mM NaCℓ, 7mM MgCℓ₂ and 6mM 2- mercaptoethanol, followed by addition of 16 units of *Asp* 718 (manufactured by Boehringer-Mannheim GMBH) and 10 units of *Pst* I to carry out digestion reaction for 2 hours at 37° C. After the thermal treatment at 65° C for 10 minutes, an approx. 1.7-kb DNA fragment was purified by AFT method.

On the other hand, approx. 2μg of the pTrS20 plasmid DNA obtained in Referential Example 4 (1) was dissolved in 30μℓ of Y-100 buffer, followed by addition of 8 units of *Pst* I and 10 units of *Nru* I (manufactured by Boehringer-Mannheim GMBH) to carry out digestion reaction for 2 hours at 37° C. After the thermal treatment at 65° C for 10 minutes, an approx. 1.5-kb DNA fragment was purified by AFT method.

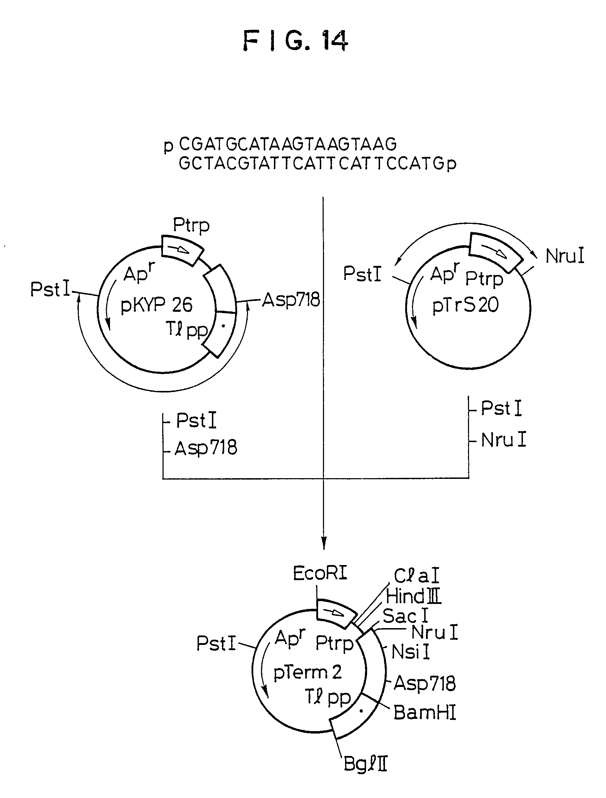

Separately, two kinds of synthetic DNAs (19 bases and 23 bases) shown in Fig. 14 were synthesized by using a 380A-DNA synthesizer (manufactured by Applied Biosystems, Inc.), which were then 5'-phosphorylated individually in the same manner as described above.

Approx. 0.1μg of the pKYP26-derived DNA fragment (approx. 1.7 kb), the pTrS20-derived DNA fragment (approx. 1.15 kb) and each 1 picomole of two kinds of 5'-phosphorylated synthetic DNAs obtained above were dissolved in 20μℓ of T4 ligase buffer, followed by addition of 50 units of T4 DNA ligase to carry out ligation reaction for 18 hours at 4° C.

Using the obtained mixture of recombinant plasmids, *Escherichia coli* strain MM294 was transformed to obtain an Ap-resistance strain. From this transformed strain, plasmid DNA pTerm2 was isolated. It was confirmed that this plasmid had the aimed structure as the result of the structural analysis by restriction enzyme digestion and the dideoxy sequencing using an M13 phage (see Fig. 14).

Referential Example 6

Construction of Recombinant Plasmid pTSF10

From cultures of *Escherichia coli* strain C600SF8 carrying plasmid ptPA7 containing human t-PA cDNA obtained in Referential Example 1, ptPA7 DNA was prepared according to an ordinary method. Approx. 2μg of the obtained ptPA7 DNA was dissolved in 30μℓ of Y-100 buffer, followed by addition of 8 units of *Bgl* II to carry out digestion reaction for 2 hours at 37° C. After the thermal treatment at 65° C for 10 minutes, an approx. 2.0-kb DNA fragment was purified by AFT method.

Then, approx. 2μg of pTrS33 DNA (Referential Example 5) was dissolved in 30μℓ of Y-100 buffer, followed by addition 10 units of *Bam* HI to carry out digestion reaction for 2 hours at 37° C. After the thermal treatment at 65° C for 10 minutes, an approx. 2.8-kb DNA fragment was purified by AFT method.

Approx. 0.1μg of the ptPA7-derived DNA fragments (approx. 2.0 kb) and approx. 0.1μg of the pTr33-derived DNA fragment (approx. 2.8 kb) obtained as described above wrre dissolved in 20μℓ (total volume) of T4 ligase buffer, followed by addition of 50 units of T4 DNA ligase to carry out ligation reaction for 18 hours at 4° C.

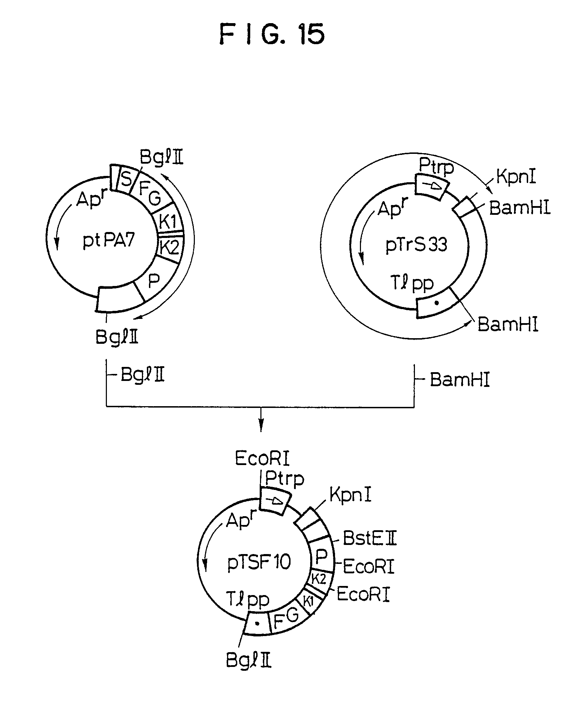

Using the obtained mixture of recombinant plasmids, *Escherichia coli* strain MM294 was transformed to obtain an Ap-resistance strain. From this strain, plasmid DNA pTSF10 was isolated. As the result of the structural analysis by restriction enzyme digestion, it was confirmed that this plasmid had the aimed structure (see Fig. 15).

Referential Example 7

Construction of Recombinant Plasmid pTA4

Approx. 3μg of pTSF10 plasmid obtained in Referential Example 6 was dissolved in 30μℓ of Y-0 buffer, followed by addition of 12 units of Kpn I to carry out digestion reaction for 2 hours at 37°C. Then, 1.5μℓ of 3M NaCℓ and 12 units of Bst EII (manufactured by New England BioLabs, Inc.) were added to the reaction solution to further carry out digestion reaction for 2 hours at 60°C. Subsequently, an aprox. 0.3-kb DNA fragment was purified by AFT method.

Separately, Escherichia coli IGHA2 (FERM BP-400) was cultured. From the cultures, pGHA2 plasmid DNA [Japanese Patent Application Kokai (Laid-open) No. 221091/1985] was prepared according to an ordinary method. Approx. 2μg of the obtained pGHA2 DNA was dissolved in 30μℓ of Y-100 buffer, followed by addition of 8 units of Pst I and 8 units of Bgl II to carry out digestion reaction for 2 hours at 37°C. After the thermal treatment at 65°C for 10 minutes, an ap-0.75-kb DNA fragment was purified by AFT method.

In addition, approx. 3μg of ptPA7 DNA (Referential Example 1) was dissolved in 30μℓ of Y-150 buffer [10mM Tris-HCℓ (pH 7.5), 7mM MgCℓ2, 6mM 2-mercaptoethanol, 150mm NaCℓ], followed by addition of 10 units of Bgl II to carry out digestion reaction for 2 hours at 37°C. Then, 12 units of Bst EII was added to the reaction solution to carry out digestion reaction for 2 hours at 60°C. Subsequently, an approx. 1.55-kb DNA fragment was purified by AFT method.

Furthermore, approx. 2μg of pTerm2 DNA obtained in Referential Example 5 was added to 30μℓ of Y-0 buffer, followed by addition of 12 units of Kpn I to carry out digestion reaction for 2 hours at 37°C. Then, 1.5μℓ of 2M Nacℓ and 8 units of Pst I were added to the reaction solution to carry out digestion reaction for 2 hours at 37°C. Subsequently, an approx. 1.7-kb DNA fragment was purified by AFT method.

Four kinds of thus obtained DNA fragments (0.03μg of the pTSF10-derived fragment, 0.05μg of the pGHA2-derived fragment, 0.1μg of the ptPA7-derived fragment and 0.1μg of the pTerm2-derived fragment) were dissolved in 2-μℓ of T4 ligase buffer, followed by addition of 200 units of T4 DNA ligase to carry out ligation reaction for 18 hours at 4°C.

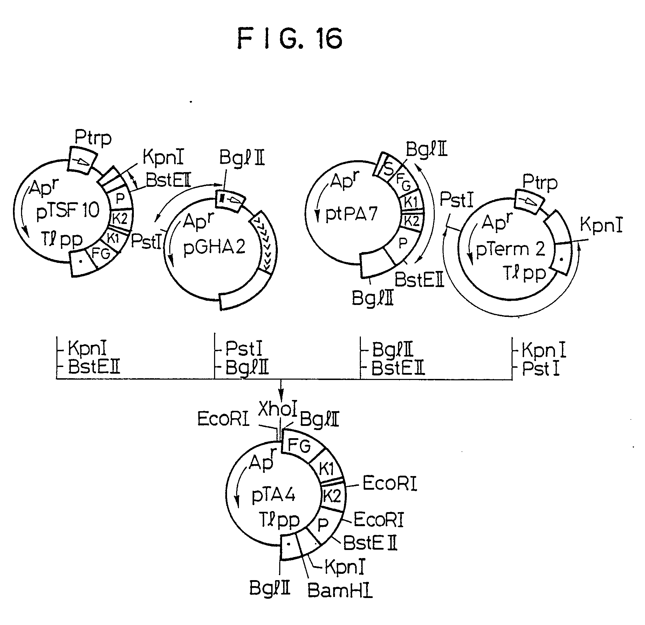

Using the obtained mixture of recombinant plasmids, Escherichia coli strain MM294 was transformed to obtain an Ap-resistance strain. From this transformed strain, plasmid DNA pTA4 was isolated. As the result of the structural analysis by restriction enzyme digestion, it was confirmed that this plasmid had the aimed structure (see Fig. 16).

Referential Example 8

Construction of t-PA-expressing Plasmid pSE1PA1-9A

(1) Construction of Recombinant Plasmid pAGE105M

Escherichia coli strain HB101 carrying plasmid pAGE 28 constructed by the present inventors [Mizukami et al, J. Biochem, vol. 101, pp. 1307~1310 (1987)] was cultured. From the culture, pAGE28 DNA was prepared according to an ordinary method. Approx. 2μg of the obtained pAGE28 DNA was dissolved in 30μℓ of Y-100 buffer, followed by addition of 8 units of Xho I and 12 units of Sca I to carry out digestion reaction for 2 hours at 37°C. After the thermal treatment at 65°C for 10 minutes, an approx. 2.8-kb DNA fragment was purified by AFT method.

On the other hand, Escherichia colistrain HB101 carrying plasmid PAGE103 constructed by the present inventors [Mizukami et al, J. Biochem, vol. 101, pp. 1307~1310 (1987)] was cultured. From the culture, pAGE103 DNA was prepared according to an ordinary method. Approx. 3μg of the obtained pAGE103 DNA was dissolved in 30μℓ of Y-100 buffer, followed by addition of 10 units of Eco RI to carry out digestion reaction for 2 hours at 37°C. After the phenol-chloroform extraction, DNA fragments were dissolved in 40μℓ (total volume) of polymerase buffer, followed by addition of 6 units of the Klenow fragment to carry out reaction for 1 hour at 15°C. Whereby, the protruding ends of Eco RI was modified to blunt ends. After stopping the reaction by phenol extraction, the reaction solution was chloroform extracted. Then, DNA frgments were recovered by ethanol precipitation therefrom. These DNA fragments were dissolved in 30μℓ of Y-100 buffer, followed by addition of 12 units of Xho I to carry out digestion reaction for 2 hours at 37°C. After the thermal treatment at 65°C for 10 minutes, an approx. 0.4-kb DNA fragment was purified by AFT method.

Furthermore, Escherichia coli strain HB101 carrying plasmid pKCR constructed by O'Hara et al [O'Hara

et al, *Proc. Natl. Acad. Sc.,* U.S.A., vol. 78, p. 1527 (1981)] was cultured. From the culture, pKCR DNA was prepared according to an ordinary method. Approx. 2μg of the obtained pKCR DNA was dissolved in 30μℓ of Y-150 buffer, followed by addition of 12 units of *Bam* HI and 16 units of *Sal* I to carry out digestion reaction for 2 hours at 37°C. After the phenol- chloroform extraction, DNA fragments were recovered by ethanol precipitation. These·DNA fragments were dissolved in 40μℓ (total volume) of polymerase buffer, followed by addition of 6 units of the Klenow fragment to carry out reaction for 1 hour at 15°C. Whereby, the protrudent ends of Baa HI and *Sal* I were modified to blunt ends. After the thermal treatment at 65°C for 10 minutes, an approx. 1.85-kb DNA fragment was purified by AFT method.

Approx. 0.05μg of the pAGE28-derived DNA fragment (approx. 2.8 kb), approx. 0.03μg of teh pAGE103-derived DNA fragment (approx. 0.4 kb) and approx. 0.2μg of the pKCR-derived DNA fragment (approx. 1.85 kb) obtained as described above were dissolved in 20μℓ of T4 ligase buffer, followed by addition of 300 units of T4 DNA ligase to carry out ligation reaction for 18 hours at 4°C.

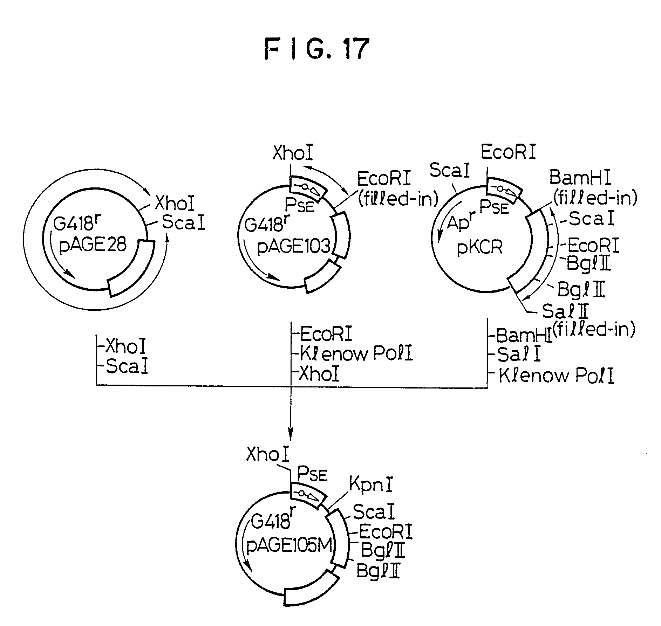

Using the obtained mixture of recombinant plasmids, *Escherichia coli* strain MM294 was transformed to obtain a kanamycin (hereinafter abbreviated to "Km")-resistance strain. From this transformed strain, plasmid pAGE105M was isolated. As a result of the structural analysis by restriction enzyme, it was confirmed that this plasmid had the aimed structure (see Fig. 17).

## (2) Construction of Recombinant Plasmid pAGE106

Approx. 2μg of pAGE105M DNA obtained above was dis solved in 30μℓ of Y-100 buffer, followed by addition of 12 units of *Sca* I to carry out digestion reaction for 2 hours at 37°C. After the thermal treatment at 65°C for 10 minutes, an approx. 5.0-kb DNA fragment was purified by AGT mehtod.

On the other hand, 5′-phosphorylated *Eco* RI linkers were prepared in the same manner as in Example 2.

Approx. 0.1μg of the pAGE105M-derived DNA fragment (approx. 5.0 kb) and 1 picomole of the 5′-phosphorylated *Eco* RI linker obtained as described above were dissolved in 20μℓ of T4 ligase buffer, followed by addition of 100 units of T4 DNA ligase to carry out ligation reaction for 18 hour at 4°C.

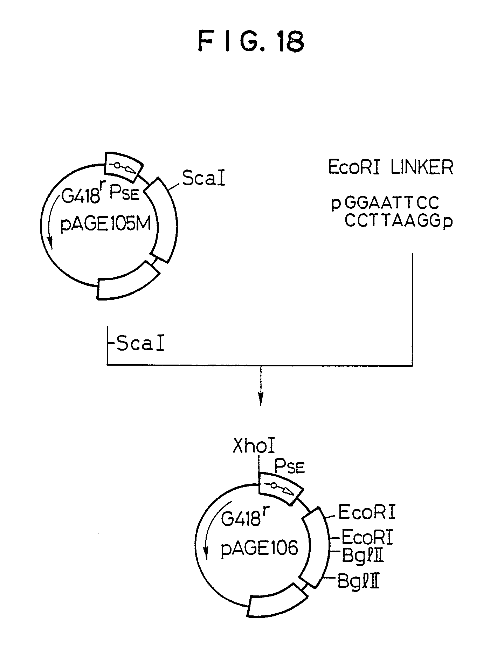

Using the obtained mixture of recombinant plasmids, *Escherichia coli* strain MM294 was transformed to obtain a Km-resistance strain. From this transformed strain, plasmid DNA pAGE 106 was isolated. As a result of the structural analysis by restriction enzyme digestion, it was confirmed that this plasmid had the aimed structure (see Fig. 18).

## (3) Construction of t-PA-expressing plasmid pSE1PA1-5

Approx. 2μg of pAGE106 DNA obtained above was dissolved in 30μℓ of Y-0 buffer, followed by addition of 12 units of *Kpn* I to carry out digestion reaction for 2 hours at 37°C. Furthermore, 1.5μℓ of 2M NaCℓ and 10 units of *Bam* HI were added to the reaction solution to carry out digestion reaction for 2 hours at 37°C. After the thermal treatment at 65°C for 10 minutes, an approx. 5.0-kb DNA fragment was purified by AFT method.

On the other hand, approx. 3μg of ptPA7 plasmid DNA obtained in Referential Example 1 was dissolved in 30μℓ of Y-0 buffer containing 75mM NaCℓ, followed by addition of each 12 units of *Fok* I and *Eco* RI to carry out digestion reaction for 2 hours at 37°C. After the thermal treatment at 65°C for 10 minutes, an approx. 0.7-kb DAN fragment was purified by AFT method.

Separately, two kinds of synthetic DNA (both 21 bases),

```
5' — GATCCATGGATGCAATGAAGA — 3'       (21 bases)
      3' — GTACCTACGTTACTTCTCTCC — 5'   (21 bases),
```

were synthesized by using a 380A-DNA synthesizer (manufactured by Applied Biosystems, Inc.), which were then 5′-phosphorylated individually in the same manner as in Example 2.

Approx. 0.1μg of the pAGE106-derived DNA fragment (approx. 5.0 kb) and approx. 0.1μg of the ptPA7-derived DNA fragment (approx. 0.7 kb) obtained as described above, approx. 0.05μg of the pTA4-derived *Eco* RI-*Kpn* I fragment (approx. 1.4 kb) prepare in Referential Example 7 and each 1 picomole of two kinds of 5′-phosphorylated synthetic DNA obtained above were dissolved in 20μℓ of T4 ligase, followed by addition of 50 units of T4 DNA ligase to carry out ligation reaction for 18hours at 4°C.

Using the obtained mixture of recombinant plasmids, *Escherichia coli* strain MM294 was transformed to obtain a Km-resistance strain. From this transformed strain, plasmid pSE1PA1-5 was isolated. As a result of the structural analysis by restriction enzyme digestion and the dideoxy sequencing using an M13 phage, it was confirmed that this plasmid had the aimed structure (see Fig. 19).

(4) Construction of t-PA-expressing plasmid pSE1PA1-9

Approx. 2μg of pSE1PA1-5 DNA obtained above was dissolved in 30μℓ of Y-0 buffer, followed by addition of 12 units of *Kpn* I to carry out digestion reaction for 2 hours at 37°C. Furthermore, 1.5μℓ of 2M NaCℓ and 8 units of *Hind* III were added to the reaction solution to carry out digestion reaction for 2 hours at 37°C. After the thermal treatment at 65°C for 10 minutes, an approx. 5.0-kb DNA fragment was purified by AFT method.

On the other hand, approx. 2μg of pSE1PA1-5 DNA was dissolved in 30μℓ of Y-0 buffer, followed by addition of 12 units of *Kpn* I to carry out digestion reaction for 2 hours at 37°C. Furthermore, 1.0μℓ of 2M NaCℓ and 10 units of *Nco* I were added to the reaction solution to carry out digestion rection for 2 hours at 37°C. After the thermal treatment at 65°C for 10 minutes, an approx. 4.9-kb DNA fragment was purified by AFT method.

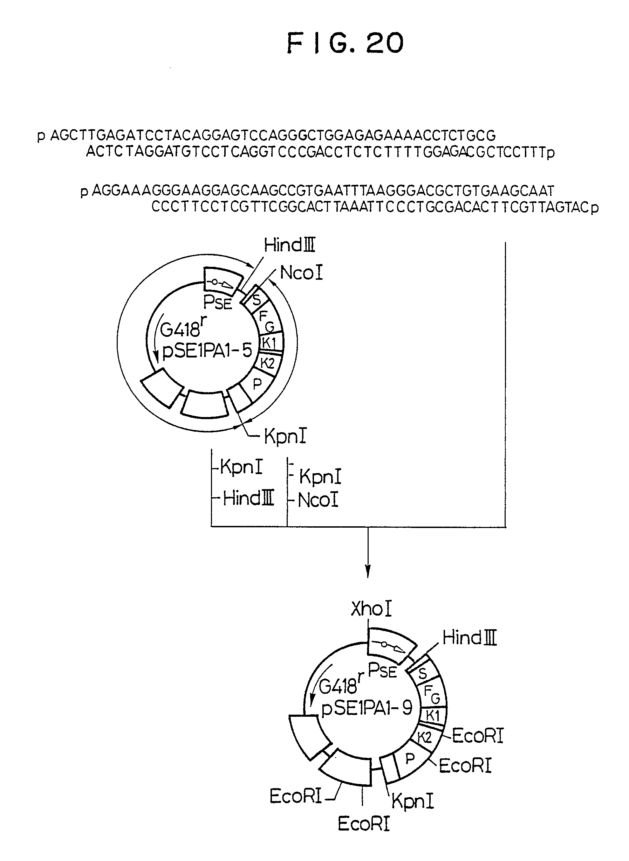

Separately, four kinds of synthetic DNA (47 bases, 49 bases, 49 bases and 47 bases; these synthetic DNAs constitute a part of 5′-non-coding region of t-PA cDNA) shown in Fig. 20,

```
                                          (47 bases)
 5'-AGCTTGAGATCCTACAGGAGTCCAGGGCTGGAGAGAAAACCTCTGCG- 3'
     3'-ACTCTAGGATGTCCTCAGGTCCCGACCTCTCTTTTGGAGACGCTCCTTT- 5'
                                          (49 bases)


                                          (49 bases)
 5'-AGGAAAGGGAAGGAGCAAGCCGTGAATTTAAGGGACGCTGTGAAGCAAT- 3'
     3'-CCCTTCCTCGTTCGGCACTTAAATTCCCTGCGACACTTCGTTAGTAC- 5'
                                          (47 bases),
```

were synthesized by using a 380A-DNA synthesizer (manufactured by Applied Biosystems, Inc.), which were then 5′-phosphorylated individually in the same manner as described in Example 2.

Each approx. 0.1μg of the pSE1PA1-5-derived approx. 5.0- and 4.9-kb DNA fragments and each 1 picomole of four kinds of 5′-phosphorylated synthetic DNA obtained as above were dissolved in 20μℓ of T4 ligase buffer, followed by addition of 50 units of T4 DNA ligase to carry out ligation reaction for 18 hours at 4°C.

Using the obtained mixture of recombinant plasmids, *Escherichia coli* strain MM294 was transformed to obtain a Km-resistance strain. From this transformed strain, plasmid DNA pSE1PA1-9 was isolated. As a result of the structural analysis by restriction enzyme digestion and the dideoxy sequencing using an M13 phage, it was confirmed that this plasmid had the aimed structure (see Fig. 20).

(5) Construction of Recombinant Plasmid pUC19H (Making Ap-resistance Gene Portable)

*Escherichia coli* strain HB101 carrying plasmid DNA constructed by Norrander et al [J. Norrander et al, *Gene*, vol. 26, p. 101 (1983); pUC19 plasmid DNA is available from Takara Shuzo Co., Ltd.] was cultured. From the culture, pUC19 DNA was prepared according to an ordianry method. Approx. 2μg of the obtained pUC19 DNA was dissolved in 30μℓ of Y-50 buffer, followed by addition of 10 units of *Hind* III and 1 unit of *Dra* I to carry out digestion reaction for 1 hour at 37°C. By this reaction, the DNA was digested completely with *Hind* III and partially with *Dra* I. After the thermal treatment at 65°C for 10 minutes, two kinds of DNA fragments, an approx. 1.55-kb *Hind* III-*Dra* I fragments and an approx. 1. 1-kb *Dra* I-*Hind* III framgment, were purified by AFT method.

Separately, 20 picomoles of *Hind* III linker (CAAGCTTG; manufactured by Colaborative Research, Inc.) was 5′-phosphorylated in the same manner as described in Example 2.

Each approx. 0.03μg of the pUC19-derived approx. 1.55-and approx. 1.1-kb fragments and 1 picomole of 5′-phosphorylated *Hind* III linker were dissolved in 20μℓ of T4 ligase buffer, followed by addition of 50 units of T4 DNA ligase to carry out ligation reaction for 18 hours at 4°C.

25

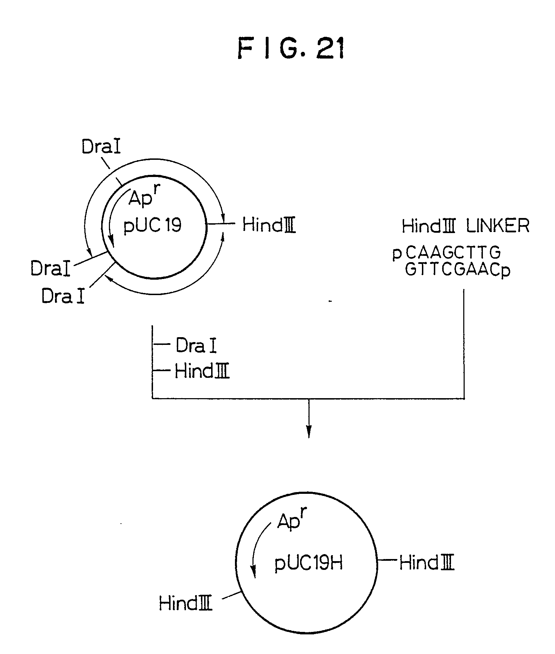

Using the obtained mixture of recombinant plasmids, *Escherichia coli* strain MM294 was transformed to obtain an Ap-resistance strain. From this transformed strain, plasmid DNA pUC19H was isolated. As a result of the structural analysis by restriction enzyme digestion, it was confirmed that this plasmid had the aimed structure (see Fig. 21).

(6) Construction of Recombinant Plasmid pSE1PA1-9A (Insertion of Ap-resistance Gene into PSE1PA1-9)

Approx. 2μg of pU619H plasmid DNA obtained above was dissolved in 30μl of Y-50 buffer, followed by addition of each 8 units of *Hind* III and *Pvu* II to carry out digestion reaction for 2 hours at 37°C. After the phenol-chloroform extraction, DNA fragments were recovered by ethanol precipitation. These DNA fragments wre dissolved in 40μl (total volume) of polymerase buffer, followed by addition of 6 units of the Klenow fragment to carry out reaction for 1 hour at 15°C. Whereby, the protruding ends of *Hind* III were modified to blunt ends. After the thermal treatment at 65°C for 10 minutes, an approx. 1.4-kb DNA fragment was purified by AFT method.

On the other hand, approx. 2μg of t-PA-expressing plasmid pSE1PA1-9 obtained above was dissolved in 30μl of Y-150 buffer, followed by addition of each 8 units of *Xho* I and *Eco* RV to carry out digestion reaction for 2 hours at 37°C. After the thermal treatment at 65°C for 10 minutes, an approx. 5.9-kb DNA fragment was purified by AFT method.

Furthermore, approx. 3μg of pAGE28 plasmid DNA prepared above was dissolved in 30μl of Y-150 buffer, followed by addition of each 10 units of *Xho* I and *Eco* RV to carry out digestion reaction for 2 hours at 37°C. After the thermal treatment at 65°C for 10 minutes, an approx. 0.85-kb DNA fragment was purified by AFT method.

Each approx. 0.1μg of the pUC19H-derived DNA fragment (approx. 1.4 kb) and the pSE1PA1-9-derived DNA fragment (approx. 5.9 kb) and approx. 0.05μg of the pAGE28-derived DNA fragment (approx. 0.85 kb) were dissolved in 20μl of T4 ligase buffer, followed by addition of 100 units of T4 DNA ligase to carry out ligation reaction for 18 hour at 4°C.

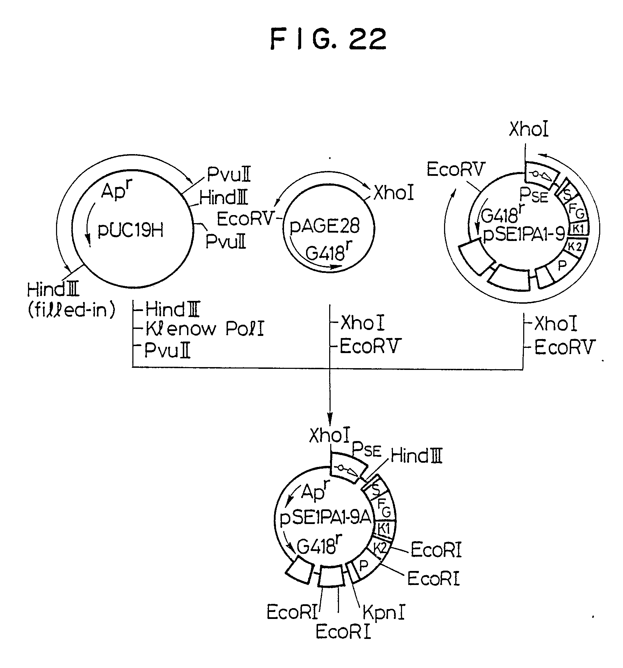

Using the obtained mixture of recombinant plasmids, *Escherichia coli* strain MM294 was transformed to obtain a strain having resistance to both Ap and Km. From this transformed strain, plasmid DNA pSE1PA1-9A was isolated. As a result of the structural analysis by restriction enzyme digestion, it was confirmed that this plasmid had the aimed structure (see Fig. 22).

A microorgaism containing the plasmid DNA pSE1PA1-9A was deposited with the Fermentation Research Institute as *Escherichia coli* EhPA1-9A (FERM BP-1460) on September 3, 1987.

Referential Example 9

Construction of Recombinant Plasmid pUKA2

From *Escherichia coli* strain C600SF8 carrying plasmid pUK1 containing human pro-UK cDNA obtained in Referential Example 2, pUK1 DNA was prepared according to an ordinary method. Approx. 2μg of the obtained pUK1 DNA was dissolved in 30μl of Y-100 buffer, followed by addition of each 8 units of *Nco* I and *Stu* I to carry out digestion reaction for 2 hours at 37°C. After the thermal treatment at 65°C for 10 minutes, an approx. 1.2-kb DNA fragment was purified by AFT method.

On the other hand, approx. 2μg of pTrS33 plasmid DNA obtained in Referential Example 4 was dissolved in solution containing 10mM Tris-HCl (pH 7.5), 25mM KCl, 7mM MgCl$_2$ and 6mM 2-mercaptoethanol (hereinafter abbreviated to "K-25 buffer"), followed by addition of 16 units of *Sma* I to carry out digestion reaction for 2 hours at 30°C. Then, 1.5μl of 1M NaCl and 10 units of *Nco* I were added to carry out the digestion reaction for 2 hours at 37°C. After the thermal treatment at 65°C for 10 minutes, an approx. 2.85-kb DNA fragment was purified by AFT method.

Approx. 0.05μg of the pUK1-derived DNA fragment (approx. 1.2 kb) and approx. 0.1μg of the pTrS33-derived DNA fragment (approx. 2.85 kb) obtained as above were dissolved in 20μl (total volume) of T4 ligase buffer, followed by ad dition of 100 units of T4 DNA ligase to carry out ligation reaction for 18 hours at 4°C.

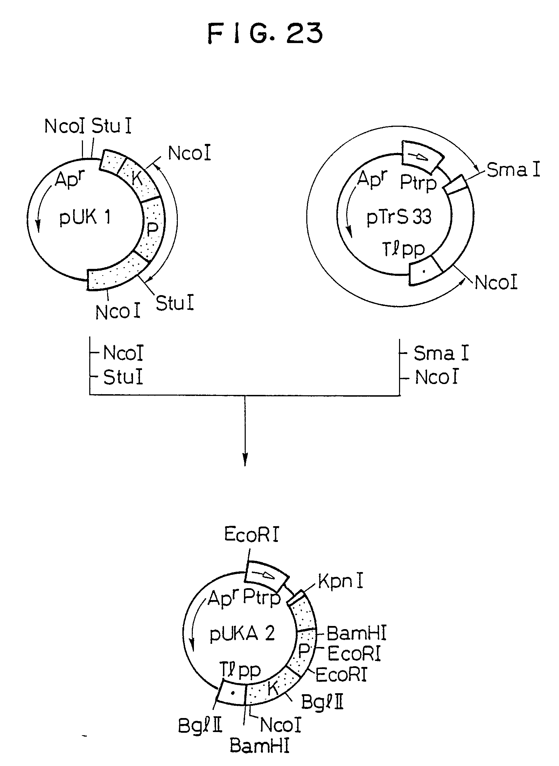

Using the obtained mixture of recombinant plasmids, *Escherichia coli* strain MM294 was transformed to obtain an Ap-resistance strain. From this transformed strain, plasmid DNA pUKA2 was isolated. As a result of the structural analysis by restriction enzyme digestion, it was confirmed that this plasmid had the aimed structure (see Fig. 23).

Referential Example 10

Construction of Human pro-UK-expressing plasmid pSE1UKpro1

(1) Construction of recombinant plasmid pUKF2

Approx. 3μg of pUK11 plasmid DNA obtained in Referential Example 3 was dissolved in 30μℓ of Y-100 buffer, followed by addition of each 12 units of *Nco* I and *Hind* III to carry out digestion reaction for 2 hours at 37°C. After the thermal treatment at 65°C for 10 minutes, an approx. 0.45-kb DNA fragment was purified by AFT method.

On the other hand, approx. 2μg of pUKA2 plasmid DNA obtained in Referential Example 9 was dissolved in 30μℓ of Y-0 buffer, followed by addition of 10 units of *Kpn* I to carry out digestion reaction for 2 hours at 37°C. Then, 1.5μℓ of 2M NaCℓ and 10 units of *Nco* I were added to the reaction solution to carry out digestion reaction for 2 hours at 37°C. After the thermal treatment at 65°C for 10 minutes, an approx. 1.2-kb DNA fragment was purified by AFT method.

Furthermore, approx. 2μg of pTerm2 plasmid obtained in Referential Example 5 was dissolved in 30μℓ of Y-0 buffer, followed by addition of 10 units of *Kpn* I to carry out digestion reaction for 2 hours at 37°C. Subsequently, 1.5μℓ of 2M NaCℓ and 8 units of *Hind* III were added to the reaction solution to carry out digestion reaction for 2 hours at 37°C. After the thermal treatment at 65°C for 10 minutes, an approx. 2.85-kb DNA fragment was purified by AFT method.

Approx. 0.2μg of the pUK11-derived DNA fragments (approx. 0.45 kb), approx. 0.05μg of the pUKA2-derived DNA fragment (approx. 1.2 kb) and approx. 0.05μg of the pTerm2-derived DNA fragment (2.85 kb) obtained as above were dissolved in 20μℓ (total volume) of T4 ligase buffer, followed by addition of 50 units of T4 DNA ligase to carry out ligation reaction for 18 hours at 4°C.

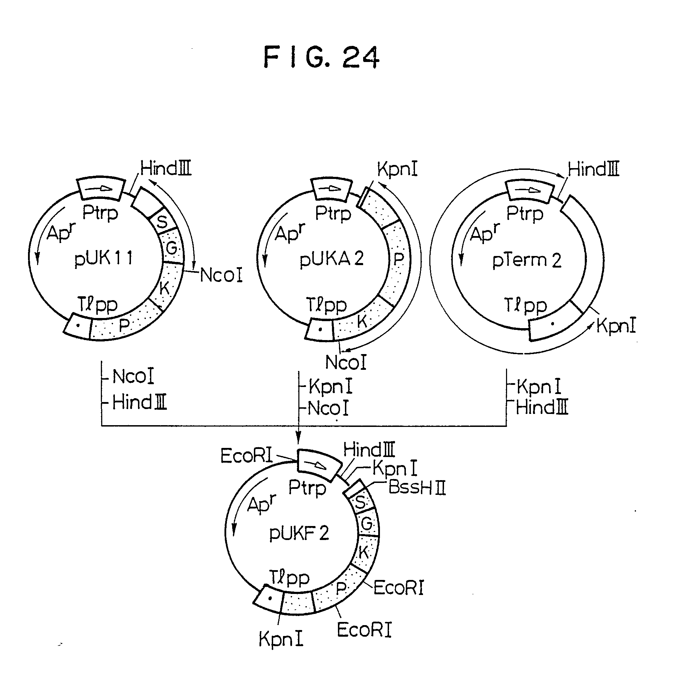

Using the obtained mixture of recombinant plasmids, *Escherichia coli* strain MM294 was transformed to obtain an Ap-resistance strain. From this transformed strain, plasmid DNA pUKF2 was isolated. As a result of the structural analysis by restriction enzyme digestion, it was confirmed that this plasmid had the aimed structure (see Fig. 24).

(2) Construction of Recombinant Plasmid pUKFpro

Approx. 2μg of pUKF2 plasmid DNA obtained above was dissolved in 30μℓ of Y-0 buffer containing 25mM NaCℓ, followed by addition of 10 units of *Bss* HII (manufactured by New England BioLabs, Inc.) to carry out digestion reaction for 2 hours at 50°C. Subsequently, 1.0μℓ of 2M NaCℓ and 10 units of *Hind* III were added to the reaction solution to carry out digestion reaction for 2 hours at 37°C. After the thermal treatment at 65°C for 10 minutes, an approx. 4.3-kb DNA fragment was purified by AFT method.

On the other hand, the following six kinds of synthetic DNA (39mer, 41mer, 41mer, 39mer, 17mer, 17mer) were synthesized and 5′-phosphorylated according to a method described above:

```
5'-AGCTTGTCCCCGCAGCGCCGTCGCGCCCTCCTGCCGCAG- 3'  (39mer)

          3'-ACAGGGGCGTCGCGGCAGCGGGCGAGGACGGCGTCCGGTGG- 5'
                                                       (41mer)

5'-GCCACCGAGGCCGCCGCCGTCTAGCGCCCCGACCTCGCCAC- 3'  (41mer)

           3'-CTCCGGCGGCGGCAGATCGCGGGGCTGGAGCGGTGGTAC- 5'  (39mer)

       5'-CATG AGA GCC CTG CTG G-3'  (17mer)

         3'-TCT CGG GAC GAC C GCGC-5'  (17mer)
```

Approx. 0.1μg of the pUKF2-derived DNA fragment (approx. 4.3 kb) and each 1 picomole of the 5'-phosphorylated six kinds of synthetic DNA obtained as above were dissolved in 20μℓ (total volume) of T4 ligase buffer, followed by addition of 300 units of T4 DNA ligase to carry out ligation reaction for 18 hours at 4°C.

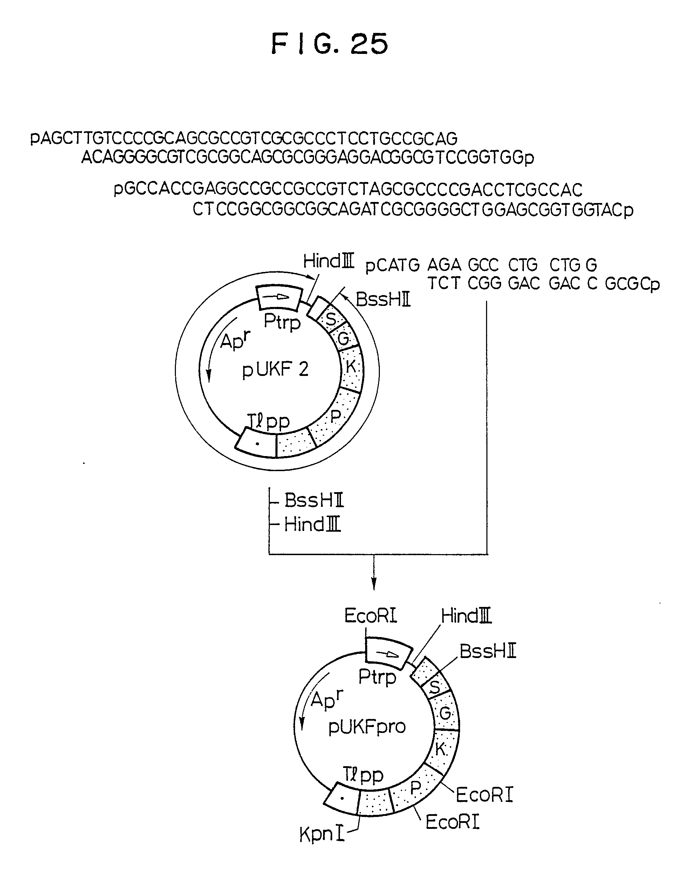

Using the obtained mixture of recombinant plasmids, *Escherichia coli* strain MM294 was transformed to obtain an Ap-resistance strain. From this strain, plasmid DNA pUKFpro was isolated. As a result of the structural analysis by restriction enzyme and the base sequence determination by M13-dideoxy sequencing, it was confirmed that this plasmid had the aimed structure (see Fig. 25).

**Claims**

1. A recombinant vector, characterized in that a promotor of bovine αS1 casein chromosomal gene and a human prourokinase gene are integrated in the vector.

2. A recombinant vector, according to claim 1, comprising a promoter of bovine αS1 casein chromosomal gene, a human prourokinase gene, a splicing signal and a poly A signal of rabbit β-globin gene and a poly A signal of SV40 integrated in the vector.

3. A recombinant vector, according to claim 1 or 2, comprising a splicing signal of a promoter of bovine αS1 casein chromosomal gene, a human prourokinase gene, a splicing signal and a poly A signal of rabbit β-globin gene, a poly A signal of SV40 and an ampicillin-resistance gene and/or G418-resistance gene integrated in the vector.

4. A recombinant vector, according to any one of the preceding claims, comprising a promoter of bovine αS1 casein chromosomal gene, a splicing signal of bovine αS1 casein chromosomal gene, a secretory signal of bovine αS1 case in chromosomal gene, a human prourokinase gene, a splicing signal and a poly A signal of rabbit β-globin gene, a poly A signal of SV40 and ampicillin-resistance gene and/or G418-resistance gene integrated in the vector.

5. A recombinant vector, according to any one of the preceding claims, wherein the promoter of bovine αS1 casein chromosomal gene is a DNA fragment contained in a fragment of approximately 6 kb gained by the digestion with Hind III and Sal I.

6. A recombinant vector, according to any one of the preceding claims, wherein the secretory signal of bovine αS1 casein chromosomal gene is Linker 1 or Linker 2 having sequences given in Formula A

```
Linker 1

Hind III              ┌Initiation -           Bovine αS1 Casein
                      │                        Signal Peptide    │
       │              │                                          │
5' - │AGCTTGACAACC  ATGAAACTTCTTATCCTCACGTGTCTTGTGGCTGTTGCTCTTGCC
3' -       ACTGTTGG  TACTTTGAAGAATAGGAGTGCACAGAACACCGACAACGAGAACGG
                     MetLysLeuLeuIleLeuThrCysLeuValAlaValAlaLeuAla

Linker 2

Hind III              ┌Initiation -           Bovine αS1 Casein
                      │                        Signal Peptide    │
       │              │                                          │
5' - │AGCTTGACAACC  ATGAAACTTCTTATCCTCACGTGTCTTGTGGCTGTTGCTCTTGCC AGG
3' -       ACTGTTGG  TACTTTGAAGAATAGGAGTGCACAGAACACCGACAACGAGAACGG TCC
                     MetLysLeuLeuIleLeuThrCysLeuValAlaValAlaLeuAla Arg


                     Formula A
```

7. A recombinant vector, according to any one of the preceding claims, wherein the human prourokinase gene is a DNA fragment containing the whole domains of signal peptide, growth factor, cringle and protease.

8. A recombinant vector, according to claim 7, wherein both ends of the human prourokinase gene carry sites resulting from digestion with Kpn I and Hind III.

28

9. An animal cell having a DNA fragment, comprising a promoter of a bovine αS1 casein chromosomal gene operably linked to a human prourokinase gene, integrated into the nucleus of the cell.

10. An animal, in which a promoter of bovine αS1 casein chromosomal gene and a human prourokinase gene are integrated in the nucleus of cells of the animal.

11. An animal, in which a promoter of bovine αS1 casein chromosomal gene and a human prourokinase gene are integrated in a chromosome in cells of the animal.

12. An animal according to claim 10 or 11, wherein the animal is a sheep, a pig, a goat, cattle or a mouse.

13. A non-human transgenic animal having a promoter of bovine αS1 casein chromosomal gene operably linked to a human prourokinase gene integrated into the nucleus of cells of the animal, or male or female progeny thereof.

14. A process for producing a non-human transgenic animal as claimed in claim 13, which comprises:

(a) introducing a DNA fragment comprising a promoter of a bovine αS1 casein chromosomal gene operably linked to a human prourokinase gene into the pronucleus of a fertilised egg, and

(b) allowing the fertilised egg to develop to a term to obtain said transgenic animal.

15. A process of producing human prourokinase, which comprises:

(a) introducing a DNA fragment containing a promoter of bovine αS1 casein chromosomal gene and human prourokinase gene into an embryo of an animal,

(b) allowing the embryo to develop to grow into a mature animal,

(c) inducing the animal or a female offspring thereof to secrete milk containing human prourokinase,

(d) collecting milk of the animal in the milk-secretion stage, and

(e) collecting human prourokinase from the milk.

16. A process according to claim 14 or 15, wherein the said DNA fragment has been obtained from plasmids pCUO, pCUW or pCUT.

17. A process according to claim 16, wherein the said DNA fragment is that of approximately 8.2 kb obtained by digesting pCUO, pCUW or pCUT with Xho I and Cla I and containing bovine αS1 casein chromosomal gene, human prourokinase gene, a splicing signal and a poly A signal of rabbit β-globin gene and a poly A signal of SV40.

18. A process of producing human prourokinase, which comprises collecting milk from a milk-producing non-human transgenic animal according to claim 13 or female progeny thereof.

19. Escherichia coli ECUO containing plasmid pCUO (FERM BP-2336), Escherichia coli ECUW containing plasmid pCUW (FERM BP-2335), Escherichia coli ECUT containing plasmid pCUT (FERM BP-2337).

20. A recombinant vector, characterised in that a promoter of bovine αS1 casein chromosomal gene and a gene which encodes a heterologous protein and which is operably linked to the promoter are integrated in the vector.

21. A non-human transgenic animal, characterised in that a promoter of bovine αS1 casein chromosomal gene and a gene which encodes a heterologous protein and which is operably linked to the promoter are integrated into the nucleus of cells of the animal, or male or female progeny thereof.

# FIG. 1

## BOVINE αS1 CASEIN CHROMOSOMAL GENE

# F I G. 2

## BOVINE αS1 CASEIN CHROMOSOMAL GENE

# F I G. 3

# F I G. 4

# FIG.5

Plasmid pSE1UKprot-2 (G418$^r$): XhoI, HindIII, A, S, G, K, P, AA, Sp, ClaI

CUTTING WITH ClaI + HindIII → FRAGMENT C

Plasmid pSE1PA1-9A (Ap$^r$, G418$^r$): XhoI, PsE, HindIII, A, S, FG, K1, K2, P, AA, Sp, ClaI

CUTTING WITH XhoI + ClaI → FRAGMENT B

Plasmid pBASIHXS (Ap$^r$): HindIII, XhoI, BglII, BglII, BglII, P$\alpha$S1

CUTTING WITH HindIII
PARTIAL CUTTING WITH BglII
T4 POLYMERASE
HindIII LINKER
CUTTING WITH HindIII + XhoI
FRAGMENT A

T4 LIGASE →

Plasmid pCUO (Ap$^r$, G418$^r$): HindIII, HUMAN PROUROKINASE cDNA, S, G, K, P, Sp, AA, ClaI, XhoI, P$\alpha$S1

FIG. 6

# FIG. 7

EP 0 390 592 A2

# F I G. 8 ( 1 )

# F I G. 8 (2)

# F I G. 9

# F I G. 10

# F I G. 11

# F I G. 12

# F I G. 13

pCGATAAGC T TATGATATCGAACGT CGACGACGGCGT CGAACCATGGCCG
TATTCGAATACTATAGCTTGCAGCTGCTGCCGCAGCTTGGTACCGGCCTAGp

# F I G. 14

p CGATGCATAAGTAAGTAAG
 GCTACGTATTCATTCATTCCATGp

# F I G. 15

# F I G. 16

# F I G. 17

# F I G. 18

# F I G. 19

pGATCCATGGATGCAATGAAGA
GTACCTACGTTACTTCTCTCCp

# F I G. 20

p AGCTTGAGATCCTACAGGAGTCCAGGGCTGGAGAGAAAACCTCTGCG
   ACTC TAGGATGTCCTCAGGT CCCGACCTC TC TT T TGGAGACGCTCCTTTp

p AGGAAAGGGAAGGAGCAAGCCGTGAATTTAAGGGACGCTGTGAAGCAAT
   CCCT TCCTCGTTCGGCACT TAAATT C CCTGCGACAC TCGTTAGTAC p

# F I G. 21

# FIG. 22

# FIG. 23

# F I G. 24

# F I G. 25

pAGCTTGTCCCCGCAGCGCCGTCGCGCCCTCCTGCCGCAG
ACAGGGGCGTCGCGGCAGCGCGGGAGGACGGCGTCCGGTGGp

pGCCACCGAGGCCGCCGCCGTCTAGCGCCCCGACCTCGCCAC
CTCCGGCGGCGGCAGATCGCGGGGGCTGGAGCGGTGGTACp

HindⅢ pCATG AGA GCC CTG CTG G
TCT CGG GAC GAC C GCGCp

BssHⅡ

Ptrp

Ap^r

pUKF 2

S
G
K
P

Tℓpp

− BssHⅡ
− HindⅢ

EcoRI    HindⅢ

BssHⅡ

Ptrp

Ap^r

pUKFpro

S
G
K
P

Tℓpp

EcoRI
EcoRI

KpnⅠ